# EUROPEAN PATENT APPLICATION

(11) **EP 4 588 485 A1**
(43) Date of publication of application: **23.07.2025**
(21) Application number: 23864803.4
(22) Date of filing: 15.09.2023
(51) Int. Cl.: A61K 45/00, A61K 31/495, A61K 31/7048, A61P 35/00

(54) **PHARMACEUTICAL COMBINATION OF FAK INHIBITOR AND TOPOISOMERASE INHIBITOR AND USE THEREOF**

(30) Priority: 16.09.2022 CN 202211129639; 07.09.2023 CN 202311155482
(71) Applicant: Inxmed (Nanjing) Co., Ltd., Nanjing, Jiangsu 210061 (CN)
(72) Inventor: ZHANG, Baoyuan, Nanjing, Jiangsu 210061 (CN); LIU, Xuebin, Nanjing, Jiangsu 210061 (CN); GAO, Jiaming, Nanjing, Jiangsu 210061 (CN); ZHANG, Ping, Nanjing, Jiangsu 210061 (CN); PANG, Ran, Nanjing, Jiangsu 210061 (CN); WANG, Zaiqi, Nanjing, Jiangsu 210061 (CN)
(74) Representative: D Young & Co LLP
(86) International application number: PCT/CN2023/119106
(87) International publication number: WO 2024/056074

(57) **Abstract**

The present disclosure relates to a combination of an FAK inhibitor, a topoisomerase inhibitor, and an immune checkpoint inhibitor for use in treating a tumor. The present disclosure also relates to a combination of an FAK inhibitor and a topoisomerase inhibitor for use in treating a tumor.

## Description

This application claims priority to Chinese patent application No. 202211129639.0 filed on September 16, 2022, and Chinese patent application No. 202311155482.3 filed on September 07, 2023. The disclosures of the above-mentioned Chinese patent applications are cited in their entirety as part of this application.

### TECHNICAL FIELD

The present disclosure belongs to the field of medicinal chemistry. Specifically, the present disclosure relates to the treatment of a tumor using a focal adhesion kinase (FAK) inhibitor in combination with other drugs.

### BACKGROUND

Tumor is the second largest killer threatening people's health. Immunogenic cell death (ICD) is based on the superposition effect of programmed cell death. An intracellular pressure signal may be activated when cancer cells are exposed to chemotherapy or targeted medicaments. This kind of pressure signal includes endoplasmic reticulum pressure (ER stress) and reactive oxygen species pressure (oxidative stress). Under the action of the pressure signal, cells will try to repair the pressure first. The cells will start the programmed death process, if the damage caused by the pressure exceeds the repair ability of the cells. This process is often accompanied by the release of a class of damage associated molecular patterns (DAMPs). This kind of DAMPs will be specifically recognized by pattern recognition receptors on antigen-presenting cells (APC) in the body, which will induce the maturation, differentiation and activation of APC, and then gradually present it to immune cells such as effector T cells, thus making immune cells generate antigen memory. When tumor cells from the same source are found again, immune cells will specifically recognize and kill the tumor cells. The new tumor-specific immune response initiated by ICD can increase the sensitivity to an immune checkpoint inhibitor (ICI), so as to enhance the effect of the immune checkpoint inhibitor, and produce anti-tumor response with lasting immune memory.

FAK, also known as protein tyrosine kinase 2 (PTK2), is a non-receptor tyrosine kinase and a key component of focal adhesion complex. FAK plays an important role in mediating integrin and growth factor signals to regulate the invasion, proliferation and survival of tumor cells.

The in vivo function of topoisomerase is to release the tension generated during DNA replication and transcription by cutting the DNA chain, to remove the positive supercoil produced at the front end of the replication and the negative supercoil generated by RNA polymerization during transcription, and then connect the cut ends formed by the cutting into a complete DNA chain to ensure the smooth progress of the reaction. Topoisomerase inhibitors bind to the easily dissociable complex of the corresponding isomerase and form a stable complex, which prevents the replication fork from passing smoothly, thereby causing the DNA chain to break, and interferes with DNA replication and transcription. This type of drug is widely used in the treatment of tumors. However, this type of drug will develop certain drug resistance after taking it for a period of time.

A combination of chemotherapy, including topoisomerase inhibitors, and immunotherapy has been widely used in clinical practice. However, the curative effect still needs to be improved, especially, how to enhance the efficacy and produce a lasting anti-tumor effect.

### Summary

One aspect of the present disclosure provides use of a FAK inhibitor, a topoisomerase inhibitor, and an immune checkpoint inhibitor in the manufacture of a medicament for treating a tumor in a subject, wherein the topoisomerase inhibitor is not an anthracycline.

Another aspect of the present disclosure provides a pharmaceutical combination product of a FAK inhibitor, a topoisomerase inhibitor, and an immune checkpoint inhibitor for use in treating a tumor in a subject, wherein the topoisomerase inhibitor is not an anthracycline.

Another aspect of the present disclosure provides a method for treating a tumor, comprising administering a therapeutically effective amount of a FAK inhibitor, a topoisomerase inhibitor, and an immune checkpoint inhibitor to a subject in need thereof, wherein the topoisomerase inhibitor is not an anthracycline.

Another aspect of the present disclosure provides a kit or a pharmaceutically acceptable composition comprising: (a) a FAK inhibitor; (b) a topoisomerase inhibitor, wherein the topoisomerase inhibitor is not an anthracycline; and (c) an immune checkpoint inhibitor.

Another aspect of the present disclosure provides use of a FAK inhibitor and a topoisomerase inhibitor in the manufacture of a medicament for treating a tumor, wherein the FAK inhibitor is used to enhance the immunogenic cell death induced by the topoisomerase inhibitor, wherein the topoisomerase inhibitor is not an anthracycline.

Another aspect of the present disclosure provides a FAK inhibitor for use in enhancing immunogenic cell death induced by a topoisomerase inhibitor in the treatment of a tumor, wherein the topoisomerase inhibitor is not an anthracycline.

Another aspect of the present disclosure provides a method for treating a tumor, comprising administering a therapeutically effective amount of a FAK inhibitor and a topoisomerase inhibitor to a subject in need thereof, wherein the FAK inhibitor is used to enhance the immunogenic cell death induced by the topoisomerase inhibitor, wherein the topoisomerase inhibitor is not an anthracycline.

Another aspect of the present disclosure provides use of a FAK inhibitor, a topoisomerase inhibitor, and an immune checkpoint inhibitor in the manufacture of a medicament for combined treatment of a tumor, wherein the topoisomerase inhibitor is not an anthracycline.

Another aspect of the present disclosure provides use of a FAK inhibitor in the manufacture of a combined medicament with a topoisomerase inhibitor and an immune checkpoint inhibitor for treating a tumor, wherein the topoisomerase inhibitor is not an anthracycline.

Another aspect of the present disclosure provides use of a topoisomerase inhibitor in the manufacture of a combined medicament with a FAK inhibitor and an immune checkpoint inhibitor for treating a tumor, wherein the topoisomerase inhibitor is not an anthracycline.

Another aspect of the present disclosure provides use of an immune checkpoint inhibitor in the manufacture of a combined medicament with a FAK inhibitor and a topoisomerase inhibitor for treating a tumor, wherein the topoisomerase inhibitor is not an anthracycline.

Another aspect of the present disclosure provides use of a FAK inhibitor in the manufacture of a medicament for combined treatment of a tumor with a topoisomerase inhibitor and an immune checkpoint inhibitor, wherein the topoisomerase inhibitor is not an anthracycline.

Another aspect of the present disclosure provides use of a topoisomerase inhibitor in the manufacture of a medicament for combined treatment of a tumor with a FAK inhibitor and an immune checkpoint inhibitor, wherein the topoisomerase inhibitor is not an anthracycline.

Another aspect of the present disclosure provides use of an immune checkpoint inhibitor in the manufacture of a medicament for combined treatment of a tumor with a FAK inhibitor and a topoisomerase inhibitor, wherein the topoisomerase inhibitor is not an anthracycline.

Another aspect of the present disclosure provides a kit, which includes: a FAK inhibitor; and an instruction, wherein the instruction indicates that the FAK inhibitor can be used in combination with a topoisomerase inhibitor and an immune checkpoint inhibitor to treat a tumor, wherein the topoisomerase inhibitor is not an anthracycline.

Another aspect of the present disclosure provides a kit, which includes: a topoisomerase inhibitor; and an instruction, wherein the instruction indicates that the topoisomerase inhibitor can be used in combination with a FAK inhibitor and an immune checkpoint inhibitor to treat a tumor, wherein the topoisomerase inhibitor is not an anthracycline.

Another aspect of the present disclosure provides a kit, which includes: an immune checkpoint inhibitor; and an instruction, wherein the instruction indicates that the immune checkpoint inhibitor can be used in combination with a FAK inhibitor and a topoisomerase inhibitor to treat a tumor, wherein the topoisomerase inhibitor is not an anthracycline.

Another aspect of the present disclosure provides a method for treating a tumor, comprising administering a therapeutically effective amount of a FAK inhibitor and a topoisomerase inhibitor to a subject in need thereof, wherein the topoisomerase inhibitor is not an anthracycline.

Another aspect of the present disclosure provides a pharmaceutical combination product of a FAK inhibitor and a topoisomerase inhibitor for use in treating a tumor in a subject in need thereof, wherein the topoisomerase inhibitor is not an anthracycline.

Another aspect of the present disclosure provides use of a FAK inhibitor and a topoisomerase inhibitor in the manufacture of a combined medicament for treating a tumor, wherein the topoisomerase inhibitor is not an anthracycline.

Another aspect of the present disclosure provides use of a FAK inhibitor in the manufacture of a combined medicament with a topoisomerase inhibitor for treating a tumor, wherein the topoisomerase inhibitor is not an anthracycline.

Another aspect of the present disclosure provides use of a topoisomerase inhibitor in the manufacture of a combined medicament with a FAK inhibitor for treating a tumor, wherein the topoisomerase inhibitor is not an anthracycline.

Another aspect of the present disclosure provides use of a FAK inhibitor and a topoisomerase inhibitor in the manufacture of a medicament for combined treatment of a tumor, wherein the topoisomerase inhibitor is not an anthracycline.

Another aspect of the present disclosure provides use of a FAK inhibitor in the manufacture of a medicament for combined treatment of a tumor with a topoisomerase inhibitor, wherein the topoisomerase inhibitor is not an anthracycline.

Another aspect of the present disclosure provides use of a topoisomerase inhibitor in the manufacture of a medicament for combined treatment of a tumor with a FAK inhibitor, wherein the topoisomerase inhibitor is not an anthracycline.

Another aspect of the present disclosure provides a kit, which includes: a FAK inhibitor; and an instruction, wherein the instruction indicates that the FAK inhibitor can be used in combination with a topoisomerase inhibitor to treat a tumor, wherein the topoisomerase inhibitor is not an anthracycline.

Another aspect of the present disclosure provides a kit, which includes: a topoisomerase inhibitor; and an instruction, wherein the instruction indicates that the topoisomerase inhibitor can be used in combination with a FAK inhibitor to treat a tumor, wherein the topoisomerase inhibitor is not an anthracycline.

Optionally, the FAK inhibitor is IN10018, Defactinib, GSK2256098, PF-00562271, VS-4718, APG-2449, AMP945, AMP886 or a pharmaceutically acceptable salt thereof, alternatively IN10018, Defactinib, AMP945, or a pharmaceutically acceptable salt thereof, yet alternatively IN10018 or a pharmaceutically acceptable salt thereof, alternatively IN10018 tartrate, and the structure of IN10018 is:

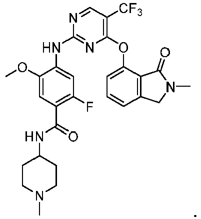

The Defactinib is also known as difatini, with a CAS number of 1345713-71-4; the CAS number of the GSK2256098 is 1224887-10-8. The CAS number of the PF-00562271 is 717907-75-0; the CAS number of the VS-4718 is 1061353-68-1; the APG-2449 is developed by Ascent Pharmaceuticals; the CAS number of the AMP945 is 1393653-34-3.

Optionally, the topoisomerase inhibitor is a topoisomerase I inhibitor.

For example, the topoisomerase I inhibitor is Topotecan, Irinotecan, Belotecan, Cositecan, Exatecan (DX-8951), Indenoisoquinoline, Indotecan (LMP-400), Indimitecan (LMP-776), Simmitecan, Gimatecan (ST1481), EC-112002, PLX-038 (NK012), AR-67, or a pharmaceutically acceptable salt thereof.

The CAS number of the Topotecan is 123948-87-8; the CAS number of the Irinotecan is 97682-44-5; the CAS number of the Belotecan is 256411-32-2; the CAS number of the Cositecan is 401905-67-7; the CAS number of the Exatecan (DX-8951) is 171335-80-1; the CAS number of the Indenoisoquinoline is 97501-75-2; the CAS number of the Indotecan (LMP-400) is 915303-09-2; the CAS number of the Indimitecan (LMP-776) is 915360-05-3; the CAS number of the Simmitecan is 951290-31-6; the CAS number of the Gimatecan (ST1481) is 292618-32-7; the EC-112002 is developed by Elucida Oncology Inc; the CAS number of the PLX-038 (NK012) is 86639-52-3; and the CAS number of the AR-67 is 220913-32-6.

Optionally, the topoisomerase I inhibitor is Irinotecan or a pharmaceutically acceptable salt thereof.

Optionally, the topoisomerase inhibitor is a topoisomerase II inhibitor.

For example, the topoisomerase II inhibitor is Etoposide, Teniposide, Sabarubicin, or a pharmaceutically acceptable salt thereof.

The CAS number of the Etoposide is 33419-42-0; the CAS number of the Teniposide is 29767-20-2; and the CAS number of the Sabarubicin is 211100-13-9.

Optionally, the topoisomerase II inhibitor is Etoposide, Teniposide, or a pharmaceutically acceptable salt thereof.

Optionally, the immune checkpoint inhibitor is an anti-PD-1/PD-L1 antibody, a PD-1/PD-L1 small molecule inhibitor or a TIGIT antibody.

Optionally, the immune checkpoint inhibitor is an anti-PD-1/PD-L1 antibody, and alternatively, the anti-PD-1/PD-L1 antibody is Pembrolizumab, Tislelizumab, Nivolumab, Toripalimab, Atezolizumab, Durvalumab, Avelumab, Atezolizumab, Camrelizumab, Sintilimab, Cemiplimab, Envafolimab, BMS-936559, JS003, SHR-1316, GS-4224, AN-4005, or MX-10181.

Optionally, the immune checkpoint inhibitor is a PD-1/PD-L1 small molecule inhibitor, and alternatively, the PD-1/PD-L1 small molecule inhibitor is INCB-086550, Lazertinib, IMMH-010, CA-170, ABSK043 or RRx-001.

Optionally, the immune checkpoint inhibitor is a TIGIT antibody, and alternatively, the TIGIT antibody is Ociperlimab (BGB-A1217), Vibostolimab, domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.

Optionally, the FAK inhibitor is IN10018 or a pharmaceutically acceptable salt thereof, the topoisomerase inhibitor is Irinotecan or a pharmaceutically acceptable salt thereof; and the immune checkpoint inhibitor is an anti-PD-1/PD-L1 antibody or a PD-1/PD-L1 small molecule inhibitor.

Optionally, the FAK inhibitor is IN10018 or a pharmaceutically acceptable salt thereof, the topoisomerase inhibitor is Irinotecan or a pharmaceutically acceptable salt thereof; and the immune checkpoint inhibitor is an anti-PD-1/PD-L1 antibody.

Optionally, the FAK inhibitor is IN10018 or a pharmaceutically acceptable salt thereof, the topoisomerase inhibitor is Etoposide, Teniposide, or a pharmaceutically acceptable salt thereof; and the immune checkpoint inhibitor is an anti-PD-1/PD-L1 antibody or a PD-1/PD-L1 small molecule inhibitor.

Optionally, the FAK inhibitor is IN10018 or a pharmaceutically acceptable salt thereof, the topoisomerase inhibitor is Etoposide, Teniposide, or a pharmaceutically acceptable salt thereof; and the immune checkpoint inhibitor is an anti-PD-1/PD-L1 antibody.

Optionally, the FAK inhibitor, the topoisomerase inhibitor and the immune checkpoint inhibitor are administered simultaneously or sequentially to the subject.

Optionally, the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian cancer, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML).

Optionally, the tumor is breast cancer or colon cancer (including colorectal cancer).

### BRIEF DESCRIPTION OF THE DRAWINGS

In order to more clearly explain the technical solution of examples of the present disclosure, the drawings of examples will be briefly introduced below. Obviously, the drawings in the following description only relate to some examples of the present disclosure, and do not limit the present disclosure.
Fig. 1 shows that FAK silencing combined with 2 µM Etoposide treatment for 48 hours increased apoptosis in MDA-MB-231 cells.
Fig. 2 shows that FAK silencing combined with 2 µM Etoposide treatment for 48 hours enhanced the release and exposure of Calreticulin.
Fig. 3 shows that FAK silencing combined with 2 µM Teniposide treatment for 48 hours increased apoptosis in MDA-MB-231 cells.
Fig. 4 shows that FAK silencing combined with 2 µM Teniposide treatment for 48 hours enhanced the release and exposure of Calreticulin.
Fig. 5 shows the IC50 value of Etoposide combined with IN10018 in colon cancer cells CT26.
Fig. 6 shows a white light microscope photograph of colon cancer CT26 cells after incubation with drugs for 48 hours.
Fig. 7 shows the situation after colon cancer CT26 cells were incubated with IN10018 and Etoposide for 48 hours, wherein: Fig. 7a shows the percentage of CRT-positive cells after colon cancer CT26 cells were incubated with drugs for 48 hours; Fig. 7b shows the percentage of Annexin V-positive cells after colon cancer CT26 cells were incubated with drugs for 48 hours.
Fig. 8 shows the situation after colon cancer CT26 cells were incubated with IN10018 and Irinotecan for 48 hours, wherein: Fig. 8a shows the percentage of CRT-positive cells after colon cancer CT26 cells were incubated with drugs for 48 hours; Fig. 8b shows the percentage of Annexin V-positive cells after colon cancer CT26 cells were incubated with drugs for 48 hours.
Fig. 9 shows the curve of the inhibitory effect of Teniposide and its combination with IN10018 on the proliferation of colon cancer CT26 cells relative to the drug concentration.
Fig. 10 shows the situation after colon cancer CT26 cells were incubated with IN10018 and Teniposide for 48 hours, wherein: Fig. 10a shows the percentage of Calreticulin (CRT)-positive cells after mouse colon cancer CT26 cells were incubated with drugs for 48 hours; Fig. 10b shows the percentage of Annexin-V positive cells after mouse colon cancer CT26 cells were incubated with drugs for 48 hours.
Fig. 11 shows the tumor growth curve of a C57BL/6 mouse subcutaneous allograft tumor model of MC38 mouse colorectal cancer cells after administration, wherein the data points represent the mean tumor volume within the group, and the error bars represent the standard error (SEM).
Fig. 12 shows the body weight change curve of a C57BL/6 mouse subcutaneous allograft tumor model of MC38 mouse colorectal cancer cells after administration, wherein the data points represent the average body weight within the group, and the error bars represent the standard error (SEM).
Fig. 13 shows the situation after mouse breast cancer T41 cells were incubated with AMP945 and Etoposide for 48 hours, wherein: Fig. 13a shows the percentage of Calreticulin (CRT)-positive cells after mouse breast cancer T41 cells were incubated with drugs for 48 hours; Fig. 13b shows the percentage of Annexin-V positive cells after mouse breast cancer T41 cells were incubated with drugs for 48 hours.
Fig. 14 shows the situation after mouse breast cancer T41 cells were incubated with AMP945 and Irinotecan for 48 hours, wherein: Fig. 14a shows the percentage of Calreticulin (CRT)-positive cells after mouse breast cancer T41 cells were incubated with drugs for 48 hours; Fig. 14b shows the percentage of Annexin-V positive cells after mouse breast cancer T41 cells were incubated with drugs for 48 hours.

### DETAILED DESCRIPTION

In order to make the purpose, technical solutions and advantages of the examples of the present disclosure more clear, the technical solution of the example of the present disclosure will be described clearly and completely with the attached Figs. Obviously, the described example is a part of examples of the present disclosure, not the whole examples. Based on the described examples of the present disclosure, all other examples obtained by ordinary people in the field without creative labor belong to the scope of protection of the present disclosure.

The present disclosure can be embodied in other specific forms without departing from the basic attributes of the present disclosure. It should be understood that any and all embodiments of the present disclosure can be combined with the technical features of any other embodiment or a plurality of other embodiments to obtain additional embodiments without conflict. The present disclosure includes further embodiments resulting from such combinations.

All publications and patents mentioned in this disclosure are hereby incorporated by reference in their entirety. If the use or terminology used in any publication and patent incorporated by reference conflicts with the use or terminology used in this disclosure, the use and terminology of the present disclosure shall prevail.

The chapter headings used in this disclosure are only for the purpose of organizing articles and should not be interpreted as limitations on the subject.

Unless otherwise specified, all technical and scientific terms used herein have the usual meaning in the field to which the claimed subject matter belongs. If there are multiple definitions of a term, the definition in the present disclosure shall prevail.

Except in working examples or otherwise indicated, all numbers of quantitative properties such as dosage stated in the description and claims should be understood as being modified in all instances by the term "about". It should also be understood that any numerical range recited in this application is intended to include all subranges within the range and any combination of endpoints of the range or subrange.

"Including", "containing", "comprising" or the like used in this disclosure means that the elements appearing before the word cover the elements listed after the word and their equivalents, but do not exclude elements that are not recorded. The term "including", "containing", or "comprising" used herein can be open, semi-closed and closed. In other words, the term also includes "consisting essentially of" or "consisting of".

### Definition

The following terms and symbols used in this application have the following meanings, unless otherwise specified in the context. As used herein, the term "FAK inhibitor" refers to an effective inhibitor of FAK, which can be suitable for mammals, alternatively humans. In some embodiments, the FAK inhibitor is IN10018, Defactinib, GSK2256098, PF-00562271, VS-4718, AMP945, AMP886 or pharmaceutically acceptable salts thereof, wherein the structure of IN10018 is 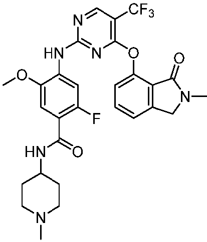 ; the Defactinib is also known as difatini, and the CAS number is 1345713-71-4; the CAS number of GSK2256098 is 1224887-10-8; the CAS number of PF-00562271 is 717907-75-0; the CAS number of VS-4718 is 1061353-68-1; the APG-2449 is developed by Yasheng Medicine; the CAS number of AMP945 is 1393653-34-3. In some embodiments, the FAK inhibitor is alternatively IN10018, Defactinib, AMP945 or a pharmaceutically acceptable salt thereof, and in some alternative embodiments, the FAK inhibitor is IN10018 or a pharmaceutically acceptable salt thereof, alternatively IN10018 tartrate.

As used herein, the term "topoisomerase inhibitor" refers to a class of compounds that can inhibit the activity of DNA topoisomerase. Common topoisomerase inhibitors include topoisomerase I inhibitors and topoisomerase II inhibitors.

As used herein, the term "anthracycline" refers to a class of topoisomerase II inhibitors, including doxorubicin, epirubicin, daunorubicin and aclarubicin, etc.

As used herein, the term "immune checkpoint inhibitor" refers to a medicament that can improve the immune system activity by regulating immune checkpoint pathways (such as PD-1, TIGIT, CTLA-4, LAG-3, TIM-3, etc.). In some embodiments, the immune checkpoint inhibitor is an antagonist of the PD-1/PD-L1 (programmed cell death protein 1) pathway (also called "PD-1 inhibitor") or a TIGIT inhibitor. PD-1 inhibitors are also referred to as PD-1/PD-L1 inhibitors in this disclosure. For example, in the therapeutic methods, medicaments and uses of the present disclosure, the PD-1/PD-L1 inhibitor is a PD-1/PD-L1 antibody, including, but not limited to, pabolizumab (KERIDA/Keytruda/K medicament), Tislelizumab (Baizean), Nivolumab, Toripalimab (Tuoyi), Atezolizumab (Tecentriq), durvalumab (Imfinzi), Avelumab (Bavencio), Atezolizumab (MPDL 3280A/Tecentriq/T medicament), BMS-936559 (fully humanized IgG4 monoclonal antibody against PD-L1), GS-4224, AN-4005 or MX-10181. In some alternative embodiments, the PD-1 inhibitor is Toripalimab. In some embodiments, PD-1 inhibitors are used to treat human subjects. In some embodiments, PD-1 is human PD-1. PD-1/PD-L1 inhibitors also include PD-1/PD-L1 small molecule inhibitors, for example INCB-086550, Lazertinib, IMMH-010, CA-170, ABSK043 or RRx-001. TIGIT (also known as WUCAM, Vstm3, VSIG9) is a receptor of the Ig superfamily and is a new immune checkpoint besides PD-1/PD-L1. For example, in the therapeutic methods, medicaments and uses of the present disclosure, the immune checkpoint inhibitor is a TIGIT inhibitor, including, but not limited to, Ociperlimab (BGB-A1217), Vibostolimab, domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321. In some embodiments, the TIGIT inhibitors are used to treat human subjects. In order to avoid ambiguity, all antibodies in the present disclosure include double antibodies.

As used herein, "pharmaceutical combination" or "pharmaceutical combination product" can refer to the case of a fixed combination in the form of a dosage unit (for example, all pharmaceutical active ingredients exist in one dosage form) or the case of a product that is administered in combination in a complete kit, and the case of a combination of a medicament and instructions indicating that the medicament can be used in combination with one or more other medicaments.

As used herein, "combined treatment" or "combined medicament" means that a medicament is used in combination with one or more other medicaments to treat a disease, including both the combination of one medicament and one or more other medicaments and the combination of one medicament and instructions indicating that the medicament can be used in combination with one or more other medicaments.

In this application, "simultaneous or sequential administration" refers to the simultaneous administration of two or more medicaments or sequential administration of two or more medicaments at a certain time interval within an administration cycle (for example, within 4 weeks, 3 weeks, 2 weeks, 1 week or 24 hours). The modes of administration (for example, oral administration, intravenous administration, intramuscular administration or subcutaneous administration) may be the same or different, and the frequency/cycle of administration of two or more medicaments may be the same or different. When the therapeutic method, product or use of the present disclosure involves two medicaments, the two medicaments can be administered simultaneously or separately at a certain interval. When the therapeutic method, product or use of the present disclosure involves three medicaments, the three medicaments can be administered at the same time point, or two medicaments can be administered at one time point and the remaining one medicament can be administered at another time point, or each of the three medicaments can be administered at different time points.

In some embodiments, the PD-1/PD-L1 inhibitor is administered intravenously (e.g., as intravenous infusion), subcutaneously or orally. Alternatively, the PD-1/PD-L1 inhibitor is administered by intravenous infusion.

In some embodiments, the TIGIT inhibitor is administered intravenously (e.g., as intravenous infusion), subcutaneously or orally. Alternatively, the TIGIT inhibitor is administered by intravenous infusion.

The ability of immune checkpoint inhibitors to treat cancer depends on the existence of tumor antigen-specific T cells in a tumor tissue. This requires tumor tissues to express antigens that distinguish themselves from their untransformed counterparts, for example, through a new protein product called neoantigen. The neoantigen load of tumor is closely related to immunogenicity and sensitivity (for example, sensitivity to checkpoint inhibitor therapy), which means that tumors with poor immunogenicity should be resistant to these medicaments to a great extent. Therapeutics for releasing tumor antigens that can be ingested by APC, such as those that induce immunogenic cell death (ICD), may promote effective anti-tumor immunity, alternatively when further combined with a checkpoint inhibitor.

As used herein, the term "treatment" refers to the administration of one or more pharmaceutical substances to a subject suffering from a disease or having symptoms of the disease in order to cure, alleviate, mitigate, change, treat, improve, ameliorate or influence the disease or symptoms of the disease. In some embodiments, the disease is a tumor or cancer.

As used herein, the term "tumor" refers to the abnormal pathological changes formed by the abnormal proliferation of clonal cells caused by the loss of normal regulation of the growth of cells of local tissues at the gene level under the action of various tumorigenic factors. The tumor includes, but is not limited to: bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian cancer, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML). In some embodiments, the tumor is breast cancer, or colon cancer (including colorectal cancer).

As used herein, the term "subject" or "patient" refers to mammals and non-mammals. Mammals refer to any member of mammals, including but not limited to: human; non-human primates, such as chimpanzees and other apes and monkey species; farm animals such as cattle, horses, sheep, goats and pigs; domestic animals such as rabbits, dogs and cats; laboratory animals, including rodents, such as rats, mice and guinea pigs, etc. Examples of non-mammals include but are not limited to birds, etc. The term "subject" is not limited to a specific age or gender. In some embodiments, the subject is a human.

As used herein, the term "pharmaceutically acceptable" means non-toxic, biologically tolerable and suitable for administration to a subject.

As used herein, the term "pharmaceutically acceptable salt" refers to non-toxic and biologically tolerable acid addition salts suitable for administration to subjects, including but not limited to: acid addition salts formed with inorganic acids, such as hydrochloride, hydrobromide, carbonate, bicarbonate, phosphate, sulfate, sulfite, nitrate, etc.; and acid addition salts with organic acids, such as formate, acetate, malate, maleate, fumarate, tartrate, succinate, citrate, lactate, methanesulfonate, p-toluenesulfonate, 2-hydroxyethyl sulfonate, benzoate, salicylate, stearate and salts formed with alkanedicarboxylic acids of formula HOOC-(CH₂)ₙ-COOH (where n is 0-4), etc.

Furthermore, a pharmaceutically acceptable acid addition salt can be obtained by dissolving a free base in a suitable solvent and treating the solution with an acid according to a conventional operation for preparing an acid addition salt from a basic compound. Those skilled in the art can determine various synthetic methods that can be used to prepare nontoxic pharmaceutically acceptable acid addition salts without redundant experiments.

As used herein, the term "pharmaceutically acceptable composition" means that it must be chemically and/or toxicologically compatible with other ingredients included in the preparation, and/or compatible with the subject to be treated. As used herein, the term "therapeutically effective amount" refers to an amount generally sufficient to produce a beneficial therapeutic effect on a subject. The therapeutically effective amount of the present disclosure can be determined by conventional methods (such as modeling, dose escalation study or clinical trial) combined with conventional influencing factors (such as administration mode, pharmacokinetics of compounds, severity and course of diseases, medical history of subjects, health status of subjects, response degree of subjects to medicaments, etc.).

As used herein, the term "inhibition" refers to a decrease in the baseline activity of a biological activity or process.

As used herein, the term "kit" refers to a box for containing chemical reagents for detecting chemical components, medicament residues, virus types and the like. The kit of the present disclosure can include (i) one, two or three of a FAK inhibitor, a topoisomerase inhibitor and an immune checkpoint inhibitor; and (ii) an instruction indicating that the FAK inhibitor, the topoisomerase inhibitor and the immune checkpoint inhibitor can be used to treat a tumor in the subject. In one embodiment, the kit includes (i) a FAK inhibitor; and (ii) an instruction indicating that the FAK inhibitor, a topoisomerase inhibitor and an immune checkpoint inhibitor can be used to treat a tumor in a subject. In one embodiment, the kit includes (i) a topoisomerase inhibitor; and (ii) an instruction indicating that a FAK inhibitor, the topoisomerase inhibitor and an immune checkpoint inhibitor can be used to treat a tumor in a subject. In one embodiment, the kit includes (i) an immune checkpoint inhibitor; and (ii) an instruction indicating that a FAK inhibitor, a topoisomerase inhibitor and the immune checkpoint inhibitor can be used to treat a tumor in a subject. In one embodiment, the kit includes (i) a FAK inhibitor, a topoisomerase inhibitor and an immune checkpoint inhibitor; and (ii) an instruction indicating that the FAK inhibitor, the topoisomerase inhibitor and the immune checkpoint inhibitor can be used to treat a tumor in a subject. In one embodiment, the kit includes (i) a FAK inhibitor; and (ii) an instruction indicating that the FAK inhibitor and a topoisomerase inhibitor can be used to treat a tumor in a subject. In one embodiment, the kit includes (i) a topoisomerase inhibitor; and (ii) an instruction indicating that the topoisomerase inhibitor and a FAK inhibitor can be used to treat a tumor in a subject.

The compounds of a kit can be contained in separate containers. Optionally, two or more compounds are contained in the same container. For example, the kit may include a first container, a second container, a third container, and a package insert, wherein the first container includes at least one dose of a medicament including a FAK inhibitor, the second container includes at least one dose of a topoisomerase inhibitor, and the third container includes at least one dose of a medicament including an immune checkpoint inhibitor, and the package insert includes instructions of treating a tumor in a subject with the medicament. The first container, the second container and the third container may contain the same or different shapes (e.g., vials, syringes and bottles) and/or materials (e.g., plastic or glass). The kit can also include other materials that can help to administer medicaments, such as diluents, filters, IV bags and lines, needles and syringes.

The exact amount of the FAK inhibitor, the topoisomerase inhibitor and the immune checkpoint inhibitor to be administered to a subject will depend on various factors, such as a given medicament or compound, pharmaceutical preparation, route of administration, type of disease, symptoms, identity of the subject or host to be treated, etc., but it can still be routinely determined by those skilled in the art. For example, determining the effective amount also depends on the degree, severity and type of cell proliferation. The skilled person will be able to determine the appropriate dosage based on these and other factors.

The FAK inhibitor, the topoisomerase inhibitor and the immune checkpoint inhibitor can be administered in an appropriate manner, such as oral administration, intravenous administration, intramuscular administration, or subcutaneous administration.

For example, when administered orally, the medicament can be administered orally together with a pharmaceutically acceptable carrier such as an inert diluent or an absorbable edible carrier. They can be encapsulated in hard-shell or soft-shell gelatin capsules, can be pressed into tablets, or can be directly mixed with patients' food. For example, medicaments can be combined with one or more excipients and used in the form of ingestible tablets, oral tablets, lozenges, capsules, elixirs, suspensions, syrups or wafers. Tablets, lozenges, pills, capsules, and the like may further include a binder, such as tragacanth gum, arabic gum, corn starch or gelatin; an excipient, such as dicalcium phosphate; a disintegrator, such as corn starch, potato starch, alginic acid, and the like; a lubricant, such as magnesium stearate; or a sweetener, such as sucrose, fructose, lactose or aspartame; or a flavoring agent.

For example, when administered intravenously or intraperitoneally by infusion or injection, the medicament solution can be prepared in water, optionally mixed with a non-toxic surfactant.

Exemplary pharmaceutical dosage forms for injection or infusion include a sterile aqueous solution, a dispersion, or a sterile powder containing an active ingredient, which is suitable for temporarily preparing a sterile injection or infusion solution or dispersion. In any case, the final dosage form should be sterile, fluid and stable under the conditions of production and storage.

Sterile injection solution can be prepared by mixing the required amount of medicaments with various other components mentioned above into a suitable solvent, and then filtering and sterilizing the mixture. For sterile powder used to prepare sterile injection solution, the alternative preparation methods can be vacuum drying and freeze drying techniques, which can produce powder of active ingredient plus any other required ingredients existing after previous sterile filtration.

The amount of the FAK inhibitor, the topoisomerase inhibitor and the immune checkpoint inhibitor required for treatment can vary not only with the specific reagents selected, but also with the route of administration, the nature of the diseases to be treated, and the age and condition of the patients, and can be finally decided by the attending physician or clinician. However, in general, the dosage can be in the range of about 0.1 to about 50mg/kg body weight per day.

The FAK inhibitor is administered in a dosage range of 5mg/day to 300 mg/day in adults. In a specific embodiment, IN10018 or a pharmaceutically acceptable salt thereof is administered at a dose of 5 mg/day to 100 mg/day in adults, for exmaple, IN10018 or a pharmaceutically acceptable salt thereof is administered at a dose of 25 mg/day to 100 mg/day in adults, and the dose is calculated as IN10018.

The topoisomerase inhibitor is administered in a dose of 1-300 mg/m² per week in adults. In a specific embodiment, Irinotecan or a pharmaceutically acceptable salt thereof is administered in a dose of 1-300 mg/m² per week in adults, and the dose is calculated as Irinotecan; Etoposide or a pharmaceutically acceptable salt thereof is administered in a dose of 1-300 mg/m² per week in adults, and the dose is calculated as Etoposide; Teniposide is administered in a dose of 1-300 mg/m² per week in adults, and the dose is calculated as Teniposide.

The immune checkpoint inhibitor is administered each time at a dose of 2-10mg/kg or 50-1200mg for adults, and once every 2 to 3 weeks. In a specific embodiment, the immune checkpoint inhibitor is administered each time at a dose of 3-10mg/kg or 100-1200mg for adults and once every two to three weeks.

Technical and scientific terms not specifically defined used herein have the meanings commonly understood by those skilled in the art to which this disclosure belongs.

In some embodiments, the present disclosure also discloses:
1. A FAK inhibitor, a topoisomerase inhibitor and an immune checkpoint inhibitor, for use in a method for treating a tumor in a subject, wherein the topoisomerase inhibitor is not an anthracycline.
2. The FAK inhibitor, the topoisomerase inhibitor and the immune checkpoint inhibitor according to embodiment 1, wherein the FAK inhibitor is IN10018, Defactinib, GSK2256098, PF-00562271, VS-4718, APG-2449, AMP945, AMP886 or a pharmaceutically acceptable salt thereof, alternatively IN10018, Defactinib, AMP945 or a pharmaceutically acceptable salt thereof, yet alternatively IN10018 or a pharmaceutically acceptable salt thereof, alternatively IN10018 tartrate, and the structure of IN10018 is: 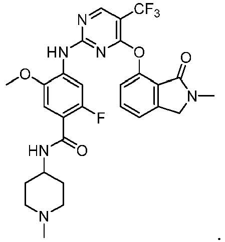
3. The FAK inhibitor, the topoisomerase inhibitor and the immune checkpoint inhibitor according to embodiment 1 or 2, wherein the topoisomerase inhibitor is a topoisomerase I inhibitor.
4. The FAK inhibitor, the topoisomerase inhibitor and the immune checkpoint inhibitor according to embodiment 3, wherein the topoisomerase I inhibitor is Topotecan, Irinotecan, Belotecan, Cositecan, Exatecan (DX-8951), Indenoisoquinoline, Indotecan (LMP-400), Indimitecan (LMP-776), Simmitecan, Gimatecan (ST1481), EC-112002 , PLX-038 (NK012), AR-67, or a pharmaceutically acceptable salt thereof.
5. The FAK inhibitor, the topoisomerase inhibitor and the immune checkpoint inhibitor according to any one of embodiments 3-4, wherein the topoisomerase I inhibitor is Irinotecan, or a pharmaceutically acceptable salt thereof.
6. The FAK inhibitor, the topoisomerase inhibitor and the immune checkpoint inhibitor according to any one of embodiments 1-2, wherein the topoisomerase inhibitor is a topoisomerase II inhibitor.
7. The FAK inhibitor, the topoisomerase inhibitor and the immune checkpoint inhibitor according to embodiment 6, wherein the topoisomerase II inhibitor is Etoposide, Teniposide, Sabarubicin, or a pharmaceutically acceptable salt thereof.
8. The FAK inhibitor, the topoisomerase inhibitor and the immune checkpoint inhibitor according to embodiment 7, wherein the topoisomerase II inhibitor is Etoposide, Teniposide, or a pharmaceutically acceptable salt thereof.
9. The FAK inhibitor, the topoisomerase inhibitor and the immune checkpoint inhibitor according to any one of embodiments 1 to 8, wherein the immune checkpoint inhibitor is an anti-PD-1/PD-L1 antibody, a PD-1/PD-L1 small molecule inhibitor or a TIGIT antibody.
10. The FAK inhibitor, the topoisomerase inhibitor and the immune checkpoint inhibitor according to any one of embodiments 1 to 9, wherein the immune checkpoint inhibitor is an anti-PD-1/PD-L1 antibody, and optionally the anti-PD-1/PD-L1 antibody is Pembrolizumab, Tislelizumab, Nivolumab, Toripalimab, Atezolizumab, Durvalumab, Avelumab, Atezolizumab, Camrelizumab, Sintilimab, Cemiplimab, Envafolimab, BMS-936559, JS003, SHR-1316, GS-4224, AN-4005, or MX-10181.
11. The FAK inhibitor, the topoisomerase inhibitor and the immune checkpoint inhibitor according to any one of embodiments 1-9, wherein the immune checkpoint inhibitor is a PD-1/PD-L1 small molecule inhibitor, and optionally the PD-1/PD-L1 small molecule inhibitor is INCB-086550, Lazertinib, IMMH-010, CA-170, ABSK043 or RRx-001.
12. The FAK inhibitor, the topoisomerase inhibitor and the immune checkpoint inhibitor according to any one of embodiments 1 to 9, wherein the immune checkpoint inhibitor is a TIGIT antibody, and optionally, the TIGIT antibody is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
13. The FAK inhibitor, the topoisomerase inhibitor and the immune checkpoint inhibitor according to embodiment 1, wherein the FAK inhibitor is IN10018 or a pharmaceutically acceptable salt thereof, the topoisomerase inhibitor is Irinotecan or a pharmaceutically acceptable salt thereof, and the immune checkpoint inhibitor is an anti-PD-1/PD-L1 antibody or a PD-1/PD-L1 small molecule inhibitor, alternatively an anti-PD-1/PD-L1 antibody.
14. The FAK inhibitor, the topoisomerase inhibitor and the immune checkpoint inhibitor according to embodiment 1, wherein the FAK inhibitor is IN 10018 or a pharmaceutically acceptable salt thereof; the topoisomerase inhibitor is Etoposide, Teniposide, or a pharmaceutically acceptable salt thereof; the immune checkpoint inhibitor is an anti-PD-1/PD-L1 antibody or a PD-1/PD-L1 small molecule inhibitor, alternatively an anti-PD-1/PD-L1 antibody.
15. The FAK inhibitor, the topoisomerase inhibitor and the immune checkpoint inhibitor according to any one of embodiments 1 to 14, wherein the FAK inhibitor, the topoisomerase inhibitor and the immune checkpoint inhibitor are administered simultaneously or sequentially to the subject.
16. The FAK inhibitor, the topoisomerase inhibitor and the immune checkpoint inhibitor according to any one of embodiments 1 to 15, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian cancer, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.
17. The FAK inhibitor, the topoisomerase inhibitor and the immune checkpoint inhibitor according to any one of embodiments 1 to 16, wherein the tumor is breast cancer, or colon cancer (including colorectal cancer).
18. A kit or a pharmaceutically acceptable composition, comprising:
   (a) a FAK inhibitor;
   (b) a topoisomerase inhibitor, wherein the topoisomerase inhibitor is not an anthracycline; and
   (c) an immune checkpoint inhibitor.
19. The kit or the composition according to embodiment 18, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945 or a pharmaceutically acceptable salt thereof, yet alternatively, the FAK inhibitor is IN10018, or a pharmaceutically acceptable salt thereof, alternatively, the FAK inhibitor is IN10018 tartrate, wherein the IN10018 has a structure of: 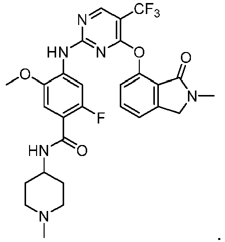
20. The kit or the composition according to any one of embodiments 18-19, wherein the topoisomerase inhibitor is a topoisomerase I inhibitor.
21. The kit or the composition according to embodiment 20, wherein the topoisomerase I inhibitor is Topotecan, Irinotecan, Belotecan, Cositecan, Exatecan (DX-8951), Indenoisoquinoline, Indotecan (LMP-400), Indimitecan (LMP-776), Simmitecan, Gimatecan (ST1481), EC-112002, PLX-038 (NK012), AR-67, or a pharmaceutically acceptable salt thereof.
22. The kit or the composition according to any one of embodiments 20-21, wherein the topoisomerase I inhibitor is Irinotecan, or a pharmaceutically acceptable salt thereof.
23. The kit or the composition according to any one of embodiments 18-19, wherein the topoisomerase inhibitor is a topoisomerase II inhibitor.
24. The kit or the composition according to embodiment 23, wherein the topoisomerase II inhibitor is Etoposide, Teniposide, Sabarubicin, or a pharmaceutically acceptable salt thereof.
25. The kit or the composition according to embodiment 24, wherein the topoisomerase II inhibitor is Etoposide, Teniposide, or a pharmaceutically acceptable salt thereof.
26. The kit or the composition according to any one of embodiments 18-25, wherein the immune checkpoint inhibitor is an anti-PD-1/PD-L1 antibody, a PD-1/PD-L1 small molecule inhibitor, or a TIGIT antibody.
27. The kit or the composition according to any one of embodiments 18-26, wherein the immune checkpoint inhibitor is an anti-PD-1/PD-L1 antibody, optionally, the anti-PD-1/PD-L1 antibody is Pembrolizumab, Tislelizumab, Nivolumab, Toripalimab, Atezolizumab, Durvalumab, Avelumab, Atezolizumab, Camrelizumab, Sintilimab, Cemiplimab, Envafolimab, BMS-936559, JS003, SHR-1316, GS-4224, AN-4005, or MX-10181.
28. The kit or the composition according to any one of embodiments 18-26, wherein the immune checkpoint inhibitor is a PD-1/PD-L1 small molecule inhibitor, optionally, the PD-1/PD-L1 small molecule inhibitor is INCB-086550, Lazertinib, IMMH-010, CA-170, ABSK043, or RRx-001.
29. The kit or the composition according to any one of embodiments 18-26, wherein the immune checkpoint inhibitor is a TIGIT antibody, optionally, the TIGIT antibody is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
30. The kit or the composition according to embodiment 18, wherein the FAK inhibitor is IN10018 or a pharmaceutically acceptable salt thereof, the topoisomerase inhibitor is Irinotecan or a pharmaceutically acceptable salt thereof; and the immune checkpoint inhibitor is an anti-PD-1/PD-L1 antibody, or a PD-1/PD-L1 small molecule inhibitor, alternatively an anti-PD-1/PD-L1 antibody.
31. The kit or the composition according to embodiment 18, wherein the FAK inhibitor is IN10018 or a pharmaceutically acceptable salt thereof, the topoisomerase inhibitor is Etoposide, Teniposide, or a pharmaceutically acceptable salt thereof, and the immune checkpoint inhibitor is an anti-PD-1/PD-L1 antibody, or a PD-1/PD-L1 small molecule inhibitor, alternatively an anti-PD-1/PD-L1 antibody.
32. The kit or the composition according to any one of embodiments 18-31, wherein the kit or the composition is used as a medicament.
33. The kit or the composition according to embodiment 32, wherein the medicament is used for treating a tumor, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian cancer, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.
34. The kit or the composition according to embodiment 33, wherein the tumor is breast cancer, or colon cancer (including colorectal cancer).
35. A method for treating a tumor in a subject, wherein the method comprises administering to the subject a therapeutically effective amount of a FAK inhibitor, a topoisomerase inhibitor and an immune checkpoint inhibitor, wherein the topoisomerase inhibitor is not an anthracycline.
36. The method according to embodiment 35, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945 or a pharmaceutically acceptable salt thereof, yet alternatively, the FAK inhibitor is IN10018, or a pharmaceutically acceptable salt thereof, alternatively, the FAK inhibitor is IN10018 tartrate, wherein the IN10018 has a structure of: 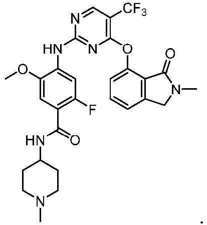
37. The method according to any one of embodiments 35-36, wherein the topoisomerase inhibitor is a topoisomerase I inhibitor.
38. The method according to embodiment 37, wherein the topoisomerase I inhibitor is Topotecan, Irinotecan, Belotecan, Cositecan, Exatecan (DX-8951), Indenoisoquinoline, Indotecan (LMP-400), Indimitecan (LMP-776), Simmitecan, Gimatecan (ST1481), EC-112002, PLX-038 (NK012), AR-67, or a pharmaceutically acceptable salt thereof.
39. The method according to embodiment 38, wherein the topoisomerase I inhibitor is Irinotecan, or a pharmaceutically acceptable salt thereof.
40. The method according to any one of embodiments 35-36, wherein the topoisomerase inhibitor is a topoisomerase II inhibitor.
41. The method according to embodiment 40, wherein the topoisomerase II inhibitor is Etoposide, Teniposide, Sabarubicin, or a pharmaceutically acceptable salt thereof.
42. The method according to embodiment 41, wherein the topoisomerase II inhibitor is Etoposide, Teniposide, or a pharmaceutically acceptable salt thereof.
43. The method according to any one of embodiments 35-42, wherein the immune checkpoint inhibitor is an anti-PD-1/PD-L1 antibody, a PD-1/PD-L1 small molecule inhibitor or a TIGIT antibody.
44. The method according to any one of embodiments 35-43, wherein the immune checkpoint inhibitor is an anti-PD-1/PD-L1 antibody, optionally, the anti-PD-1/PD-L1 antibody is Pembrolizumab, Tislelizumab, Nivolumab, Toripalimab, Atezolizumab, Durvalumab, Avelumab, Atezolizumab, Camrelizumab, Sintilimab, Cemiplimab, Envafolimab, BMS-936559, JS003, SHR-1316, GS-4224, AN-4005, or MX-10181.
45. The method according to any one of embodiments 35-43, wherein the immune checkpoint inhibitor is a PD-1/PD-L1 small molecule inhibitor, optionally, the PD-1/PD-L1 small molecule inhibitor is INCB-086550, Lazertinib, IMMH-010, CA-170, ABSK043, or RRx-001.
46. The method according to any one of embodiments 35-43, wherein the immune checkpoint inhibitor is a TIGIT antibody, optionally, the TIGIT antibody is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
47. The method according to embodiment 35, wherein the FAK inhibitor is IN10018 or a pharmaceutically acceptable salt thereof, the topoisomerase inhibitor is Irinotecan or a pharmaceutically acceptable salt thereof; and the immune checkpoint inhibitor is an anti-PD-1/PD-L1 antibody, or a PD-1/PD-L1 small molecule inhibitor, alternatively an anti-PD-1/PD-L1 antibody.
48. The method according to embodiment 35, wherein the FAK inhibitor is IN10018 or a pharmaceutically acceptable salt thereof, the topoisomerase inhibitor is Etoposide, Teniposide, or a pharmaceutically acceptable salt thereof, and the immune checkpoint inhibitor is an anti-PD-1/PD-L1 antibody, or a PD-1/PD-L1 small molecule inhibitor, alternatively an anti-PD-1/PD-L1 antibody.
49. The method according to any one of embodiments 35-48, wherein the FAK inhibitor, the topoisomerase inhibitor and the immune checkpoint inhibitor are administered simultaneously or sequentially to the subject.
50. The method according to any one of embodiments 35-49, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian cancer, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.
51. The method according to any one of embodiments 35-50, wherein the tumor is breast cancer, or colon cancer (including colorectal cancer).
52. A FAK inhibitor, a topoisomerase inhibitor and an immune checkpoint inhibitor, for use in a method of treating a tumor by increasing immunogenic cell death in a subject, wherein the topoisomerase inhibitor is not an anthracycline.
53. The FAK inhibitor, the topoisomerase inhibitor and the immune checkpoint inhibitor according to embodiment 52, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, yet alternatively, the FAK inhibitor is IN10018, or a pharmaceutically acceptable salt thereof, alternatively, the FAK inhibitor is IN10018 tartrate, wherein the IN10018 has a structure of: 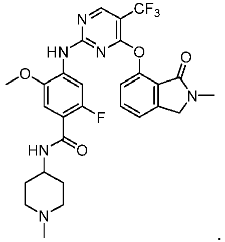
54. The FAK inhibitor, the topoisomerase inhibitor and the immune checkpoint inhibitor according to embodiments 52-53, wherein the topoisomerase inhibitor is a topoisomerase I inhibitor.
55. The FAK inhibitor, the topoisomerase inhibitor and the immune checkpoint inhibitor according to embodiment 54, wherein the topoisomerase I inhibitor is Topotecan, Irinotecan, Belotecan, Cositecan, Exatecan (DX-8951), Indenoisoquinoline, Indotecan (LMP-400), Indimitecan (LMP-776), Simmitecan, Gimatecan (ST1481), EC-112002, PLX-038 (NK012), AR-67, or a pharmaceutically acceptable salt thereof.
56. The FAK inhibitor, the topoisomerase inhibitor and the immune checkpoint inhibitor according to embodiment 55, wherein the topoisomerase I inhibitor is Irinotecan or a pharmaceutically acceptable salt thereof.
57. The FAK inhibitor, the topoisomerase inhibitor and the immune checkpoint inhibitor according to any one of embodiments 52-53, wherein the topoisomerase inhibitor is a topoisomerase II inhibitor.
58. The FAK inhibitor, the topoisomerase inhibitor and the immune checkpoint inhibitor according to embodiment 57, wherein the topoisomerase II inhibitor is Etoposide, Teniposide, Sabarubicin, or a pharmaceutically acceptable salt thereof.
59. The FAK inhibitor, the topoisomerase inhibitor and the immune checkpoint inhibitor according to embodiment 58, wherein the topoisomerase II inhibitor is Etoposide, Teniposide, or a pharmaceutically acceptable salt thereof.
60. The FAK inhibitor, the topoisomerase inhibitor and the immune checkpoint inhibitor according to any one of embodiments 52-59, wherein the immune checkpoint inhibitor is an anti-PD-1/PD-L1 antibody, a PD-1/PD-L1 small molecule inhibitor or a TIGIT antibody.
61. The FAK inhibitor, the topoisomerase inhibitor and the immune checkpoint inhibitor according to any one of embodiments 52-60, wherein the immune checkpoint inhibitor is an anti-PD-1/PD-L1 antibody, optionally, the anti-PD-1/PD-L1 antibody is Pembrolizumab, Tislelizumab, Nivolumab, Toripalimab, Atezolizumab, Durvalumab, Avelumab, Atezolizumab, Camrelizumab, Sintilimab, Cemiplimab, Envafolimab, BMS-936559, JS003, SHR-1316, GS-4224, AN-4005, or MX-10181.
62. The FAK inhibitor, the topoisomerase inhibitor and the immune checkpoint inhibitor according to any one of embodiments 52-60, wherein the immune checkpoint inhibitor is a PD-1/PD-L1 small molecule inhibitor, optionally, the PD-1/PD-L1 small molecule inhibitor is INCB-086550, Lazertinib, IMMH-010, CA-170, ABSK043, or RRx-001.
63. The FAK inhibitor, the topoisomerase inhibitor and the immune checkpoint inhibitor according to any one of embodiments 52-60, wherein the immune checkpoint inhibitor is a TIGIT antibody, optionally, the TIGIT antibody is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
64. The FAK inhibitor, the topoisomerase inhibitor and the immune checkpoint inhibitor according to embodiment 52, wherein the FAK inhibitor is IN10018 or a pharmaceutically acceptable salt thereof, the topoisomerase inhibitor is Irinotecan or a pharmaceutically acceptable salt thereof; and the immune checkpoint inhibitor is an anti-PD-1/PD-L1 antibody, or a PD-1/PD-L1 small molecule inhibitor, alternatively an anti-PD-1/PD-L1 antibody.
65. The FAK inhibitor, the topoisomerase inhibitor and the immune checkpoint inhibitor according to embodiment 52, wherein the FAK inhibitor is IN10018 or a pharmaceutically acceptable salt thereof, the topoisomerase inhibitor is Etoposide, Teniposide, or a pharmaceutically acceptable salt thereof, and the immune checkpoint inhibitor is an anti-PD-1/PD-L1 antibody, or a PD-1/PD-L1 small molecule inhibitor, alternatively an anti-PD-1/PD-L1 antibody.
66. The FAK inhibitor, the topoisomerase inhibitor and the immune checkpoint inhibitor according to any one of embodiments 52-65, wherein the FAK inhibitor, the topoisomerase inhibitor and the immune checkpoint inhibitor are administered simultaneously or sequentially to the subject.
67. The FAK inhibitor, the topoisomerase inhibitor and the immune checkpoint inhibitor according to any one of embodiments 52-66, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian cancer, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.
68. The FAK inhibitor, the topoisomerase inhibitor and the immune checkpoint inhibitor according to any one of embodiments 52-67, wherein the tumor is breast cancer, or colon cancer (including colorectal cancer).
69. A method for treating a tumor by increasing immunogenic cell death in a subject, wherein the method comprises administering to the subject a therapeutically effective amount of a FAK inhibitor, a topoisomerase inhibitor and an immune checkpoint inhibitor, wherein the topoisomerase inhibitor is not an anthracycline.
70. The method according to embodiment 69, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, yet alternatively, the FAK inhibitor is IN10018, or a pharmaceutically acceptable salt thereof, alternatively, the FAK inhibitor is IN10018 tartrate, wherein the IN10018 has a structure of: 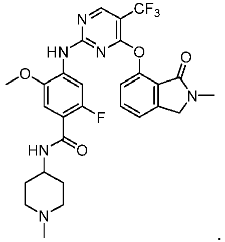
71. The method according to any one of embodiments 69-70, wherein the topoisomerase inhibitor is a topoisomerase I inhibitor.
72. The method according to any one of embodiments 69-71, wherein the topoisomerase I inhibitor is Topotecan, Irinotecan, Belotecan, Cositecan, Exatecan (DX-8951), Indenoisoquinoline, Indotecan (LMP -400), Indimitecan (LMP-776), Simmitecan, Gimatecan (ST1481), EC-112002, PLX-038 (NK012), AR-67, or a pharmaceutically acceptable salt thereof.
73. The method of embodiment 72, wherein the topoisomerase I inhibitor is Irinotecan, or a pharmaceutically acceptable salt thereof.
74. The method according to any one of embodiments 69-70, wherein the topoisomerase inhibitor is a topoisomerase II inhibitor.
75. The method according to embodiment 74, wherein the topoisomerase II inhibitor is Etoposide, Teniposide, Sabarubicin, or a pharmaceutically acceptable salt thereof.
76. The method of embodiment 75, wherein the topoisomerase II inhibitor is Etoposide, Teniposide, or a pharmaceutically acceptable salt thereof.
77. The method according to any one of embodiments 69-76, wherein the immune checkpoint inhibitor is an anti-PD-1/PD-L1 antibody, a PD-1/PD-L1 small molecule inhibitor, or a TIGIT antibody.
78. The method according to any one of embodiments 69-77, wherein the immune checkpoint inhibitor is an anti-PD-1/PD-L1 antibody, optionally, the anti-PD-1/PD-L1 antibody is Pembrolizumab, Tislelizumab, Nivolumab, Toripalimab, Atezolizumab, Durvalumab, Avelumab, Atezolizumab, Camrelizumab, Sintilimab, Cemiplimab, Envafolimab, BMS-936559, JS003, SHR-1316, GS-4224, AN-4005, or MX-10181.
79. The method according to any one of embodiments 69-77, wherein the immune checkpoint inhibitor is a PD-1/PD-L1 small molecule inhibitor, optionally, the PD-1/PD-L1 small molecule inhibitor is INCB-086550, Lazertinib, IMMH-010, CA-170, ABSK043, or RRx-001.
80. The method according to any one of embodiments 69-77, wherein the immune checkpoint inhibitor is a TIGIT antibody, optionally, the TIGIT antibody is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
81. The method according to embodiment 69, wherein the FAK inhibitor is IN10018 or a pharmaceutically acceptable salt thereof, the topoisomerase inhibitor is Irinotecan or a pharmaceutically acceptable salt thereof; and the immune checkpoint inhibitor is an anti-PD-1/PD-L1 antibody, or a PD-1/PD-L1 small molecule inhibitor, alternatively an anti-PD-1/PD-L1 antibody.
82. The method according to embodiment 69, wherein the FAK inhibitor is IN10018 or a pharmaceutically acceptable salt thereof, the topoisomerase inhibitor is Etoposide, Teniposide, or a pharmaceutically acceptable salt thereof, and the immune checkpoint inhibitor is an anti-PD-1/PD-L1 antibody, or a PD-1/PD-L1 small molecule inhibitor, alternatively an anti-PD-1/PD-L1 antibody.
83. The method according to any one of embodiments 69-82, wherein the FAK inhibitor, the topoisomerase inhibitor, and the immune checkpoint inhibitor are administered simultaneously or sequentially to the subject.
84. The method according to any one of embodiments 69-83, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian cancer, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.
85. The method according to any one of embodiments 69-84, wherein the tumor is breast cancer, or colon cancer (including colorectal cancer).
86. Use of a FAK inhibitor in the manufacture of a medicament for treating a tumor in a subject, wherein the FAK inhibitor, a topoisomerase inhibitor and an immune checkpoint inhibitor are administered to the subject, wherein the topoisomerase inhibitor is not an anthracycline.
87. Use of a topoisomerase inhibitor in the manufacture of a medicament for treating a tumor in a subject, wherein a FAK inhibitor, the topoisomerase inhibitor and an immune checkpoint inhibitor are administered to the subject, wherein the topoisomerase inhibitor is not an anthracycline.
88. Use of an immune checkpoint inhibitor in the manufacture of a medicament for treating a tumor in a subject, wherein a FAK inhibitor, a topoisomerase inhibitor, and the immune checkpoint inhibitor are administered to the subject, wherein the topoisomerase inhibitor is not an anthracycline.
89. Use of a FAK inhibitor, a topoisomerase inhibitor and an immune checkpoint inhibitor in the manufacture of a combined medicament for treating a tumor, wherein the topoisomerase inhibitor is not an anthracycline.
90. Use of a FAK inhibitor in the manufacture of a combined medicament with a topoisomerase inhibitor and an immune checkpoint inhibitor for treating a tumor, wherein the topoisomerase inhibitor is not an anthracycline.
91. Use of a topoisomerase inhibitor in the manufacture of a combined medicament with a FAK inhibitor and an immune checkpoint inhibitor for treating a tumor, wherein the topoisomerase inhibitor is not an anthracycline.
92. Use of an immune checkpoint inhibitor in the manufacture of a combined medicament with a FAK inhibitor and a topoisomerase inhibitor for treating a tumor, wherein the topoisomerase inhibitor is not an anthracycline.
93. Use of a FAK inhibitor, a topoisomerase inhibitor and an immune checkpoint inhibitor in the manufacture of a medicament for combined treatment of a tumor, wherein the topoisomerase inhibitor is not an anthracycline.
94. Use of a FAK inhibitor in the manufacture of a medicament for combined treatment of a tumor with a topoisomerase inhibitor and an immune checkpoint inhibitor, wherein the topoisomerase inhibitor is not an anthracycline.
95. Use of a topoisomerase inhibitor in the manufacture of a medicament for combined treatment of a tumor with a FAK inhibitor and an immune checkpoint inhibitor, wherein the topoisomerase inhibitor is not an anthracycline.
96. Use of an immune checkpoint inhibitor in the manufacture of a medicament for combined treatment of a tumor with a FAK inhibitor and a topoisomerase inhibitor, wherein the topoisomerase inhibitor is not an anthracycline.
97. The use according to any one of embodiments 86-96, wherein the FAK inhibitor is IN10018, Defactinib, GSK2256098, PF-00562271, APG-2449, VS-4718, AMP945, AMP886 or a pharmaceutically acceptable salt thereof, alternatively IN10018, Defactinib, AMP945 or a pharmaceutically acceptable salt thereof, yet alternatively IN10018 or a pharmaceutically acceptable salt thereof, alternatively IN10018 tartrate, wherein the structure of IN10018 is: 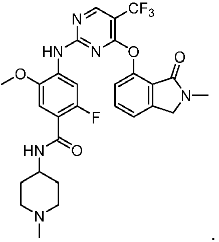
98. The use according to any one of embodiments 86-97, wherein the topoisomerase inhibitor is a topoisomerase I inhibitor.
99. The use according to embodiment 98, wherein the topoisomerase I inhibitor is Topotecan, Irinotecan, Belotecan, Cositecan, Exatecan (DX-8951), Indenoisoquinoline, Indotecan (LMP-400), Indimitecan (LMP-776), Simmitecan, Gimatecan (ST1481), EC-112002, PLX-038 (NK012), AR-67, or a pharmaceutically acceptable salt thereof.
100. The use according to embodiment 99, wherein the topoisomerase I inhibitor is Irinotecan or a pharmaceutically acceptable salt thereof.
101. The use according to any one of embodiments 86-97, wherein the topoisomerase inhibitor is a topoisomerase II inhibitor.
102. The use according to embodiment 101, wherein the topoisomerase II inhibitor is Etoposide, Teniposide, Sabarubicin, or a pharmaceutically acceptable salt thereof.
103. The use according to embodiment 102, wherein the topoisomerase II inhibitor is Etoposide, Teniposide, or a pharmaceutically acceptable salt thereof.
104. The use according to any one of embodiments 86-103, wherein the immune checkpoint inhibitor is an anti-PD-1/PD-L1 antibody, a PD-1/PD-L1 small molecule inhibitor or a TIGIT antibody.
105. The use according to any one of embodiments 86-104, wherein the immune checkpoint inhibitor is an anti-PD-1/PD-L1 antibody, and optionally the anti-PD-1/PD-L1 antibody is Pembrolizumab, Tislelizumab, Nivolumab, Toripalimab, Atezolizumab, Durvalumab, Avelumab, Atezolizumab, Camrelizumab, Sintilimab, Cemiplimab, Envafolimab, BMS-936559, JS003, SHR-1316, GS-4224, AN-4005, or MX-10181.
106. The use according to any one of embodiments 86-104, wherein the immune checkpoint inhibitor is a PD-1/PD-L1 small molecule inhibitor, and optionally the PD-1/PD-L1 small molecule inhibitor is INCB-086550, Lazertinib, IMMH-010, CA-170, ABSK043 or RRx-001.
107. The use according to any one of embodiments 86-104, wherein the immune checkpoint inhibitor is a TIGIT antibody, and optionally, the TIGIT antibody is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321.
108. The use according to any one of embodiments 86-96, wherein the FAK inhibitor is IN10018 or a pharmaceutically acceptable salt thereof, the topoisomerase inhibitor is Irinotecan or a pharmaceutically acceptable salt thereof, and the immune checkpoint inhibitor is an anti-PD-1/PD-L1 antibody or a PD-1/PD-L1 small molecule inhibitor, alternatively an anti-PD-1/PD-L1 antibody.
109. The use according to any one of embodiments 86-96, wherein the FAK inhibitor is IN10018 or a pharmaceutically acceptable salt thereof, the topoisomerase inhibitor is Etoposide, Teniposide, or a pharmaceutically acceptable salt thereof, and the immune checkpoint inhibitor is an anti-PD-1/PD-L1 antibody or a PD-1/PD-L1 small molecule inhibitor, alternatively an anti-PD-1/PD-L1 antibody.
110. The use according to any one of embodiments 86-109, wherein the FAK inhibitor, the topoisomerase inhibitor and the immune checkpoint inhibitor are administered simultaneously or sequentially to the subject.
111. The use according to any one of embodiments 86-110, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian cancer, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma, or pancreatic cancer.
112. The use according to any one of embodiments 86-111, wherein the tumor is breast cancer, or colon cancer (including colorectal cancer).

### Example

The following examples are provided to further illustrate the present disclosure. It should be understood that these examples are only used to illustrate the present disclosure and are not used to limit the scope of the present disclosure.

The assay methods without specific conditions in the following examples can be carried out according to the conventional conditions of this kind of reaction or according to the conditions suggested by the manufacturer.

Unless otherwise specified, the assay materials and reagents used in the following examples can be obtained from commercial channels.

The abbreviations used in the examples have the following meanings:

| Abbreviation | Name |
|---|---|
| siRNA | Small interfering RNA |
| DMSO | Dimethyl sulfoxide |
| COMBO | Combination |
| HMGB1 | High mobility group box-1 protein |
| AAALAC | Association for Assessment and Accreditation of Laboratory Animal Care |
| IACUC | Institutional Animal Care and Use Committee |
| SPF | Specific pathogen Free |
| BIW | Twice a week |
| QD | Once a day |
| BID | Twice a day |
| BW | Body weight |
| EDTA | Ethylenediamine tetraacetic acid |
| GLP | Good Laboratory Practice for Non-clinical Laboratory Studies |
| *p.o.* | Intragastric administration |
| *i.v.* | Intravenous injection administration |
| *i.p.* | Intraperitoneal injection administration |
| DPBS | Dulbecco's Phosphate Buffered Saline |
| DDH2O | Double distilled water |
| RT | Room temperature |
| SEM | Standard error of the mean |
| TGI | Tumor growth inhibition rate |
| TV | Tumor volume |
| TW | Tumor weight |
| FBS | Fetal bovine serum |
| PBS | Phosphate-buffered saline |
| MC | Methylcellulose |
| CO₂ | Carbon dioxide |
| CRT | Calreticulin |

| Annexin-V-FITC | Fluorescein isothiocyanate-labeled annexin |
|---|---|
| PI | Propidium iodide |

### Example 1: Study on the enhancement of immunogenic cell death of breast cancer cells in response to Etoposide by FAK target inhibition

### Assay protocol:

### 1. FAK silencing combined with Etoposide promotes apoptosis of breast cancer cells MDA-MB-231

The siRNA technology was used in the in vitro assay to reduce the expression level of FAK, and combined with Etoposide for 48 hours; flow cytometry was used to detect apoptosis in control and FAK silenced cells.

### 2. FAK silencing combined with Etoposide can effectively promote the production of ICD in breast cancer cells MDA-MB-231

The siRNA technology was used in the in vitro assay to reduce the expression level of FAK, and combined with Etoposide for 48 hours. Flow cytometry was used to detect the expression of Calreticulin, the main target of ICD.

Assay antibodies: recombinant Alexa Fluor^{®} 647 fluorescent Anti-Calreticulin antibody (Abcam, ab196159), Annexin V apoptosis detection kit (Invitrogen, A35110).

FAK siRNA was provided by Gene Pharma, and the sequence is shown in the following table:
F: CCUGUAUGCCUAUCAGCUUTT;
R: AAGCUGAUAGGCAUACAGGTT

### Assay instruments:

Fluorescence microscope (Olympus U-HGLGPS). Chemiluminescence imager (BIORAD chemidoc touch)

### Assay results:

### 1. Combining FAK silencing with Etoposide significantly increased the apoptosis of MDA-MB-231 cells

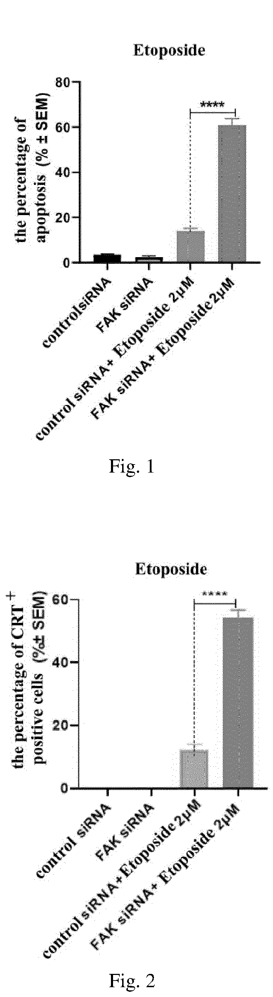

MDA-MB-231 cells were transfected with control siRNA or FAK siRNA at a final concentration of 50 nM, respectively. After the transfection for 24 hours, the cells were treated with 2 µM of Etoposide for 48 hours. The cells were stained using Annexin V kit and detected by flow cytometry. The early and late apoptotic values were counted, compared with those of the DMSO control group, and plotted using Graphpad 8.0. The results showed that compared with the control group, the apoptosis of the FAK silencing group was significantly enhanced after Etoposide treatment, as shown in Fig. 1.

### 2. Combining FAK silencing with Etoposide significantly increased the release and exposure of Calreticulin, the ICD target

MDA-MB-231 cells were transfected with control siRNA or FAK siRNA at a final concentration of 50 nM, respectively. After the transfection for 24 hours, the cells were treated with 2 µM of Etoposide for 48 hours. The cells were fluorescent stained with Calreticulin antibody, and the staining results were analyzed by flow cytometry. FlowJo software result statistics showed that after FAK silencing combined with Etoposide, the release and exposure of Calreticulin were significantly enhanced, as shown in Fig. 2.

### Example 2: Study on the enhancement of immunogenic cell death of breast cancer cells in response to Teniposide by FAK target inhibition

### Assay protocol:

### 1. FAK silencing combined with Teniposide promotes apoptosis of breast cancer cells MDA-MB-231

The siRNA technology was used in the in vitro assays to reduce the expression level of FAK, and combined with Teniposide for 48 hours; flow cytometry was used to detect apoptosis in control and FAK silenced cells.

### 2. FAK silencing combined with Teniposide can effectively promote the production of ICD in breast cancer cells MDA-MB-231

The siRNA technology was used in the in vitro assay to reduce the expression level of FAK, and combined with Teniposide for 48 hours. Flow cytometry was used to detect the expression of Calreticulin, the main target of ICD.

### Assay antibody:

Recombinant Alexa Fluor^{®} 647 fluorescent Anti-Calreticulin antibody (Abcam, ab196159), Annexin V apoptosis detection kit (Invitrogen, A35110).

FAK siRNA was provided by Gene Pharma, and the sequence is shown in the following table:
F: CCUGUAUGCCUAUCAGCUUTT;
R: AAGCUGAUAGGCAUACAGGTT

### Assay instruments:

Fluorescence microscope (Olympus U-HGLGPS). Chemiluminescence imager (BIORAD chemidoc touch).

### Assay results:

### 1. Combining FAK silencing with Teniposide significantly increased the apoptosis of MDA-MB-231 cells

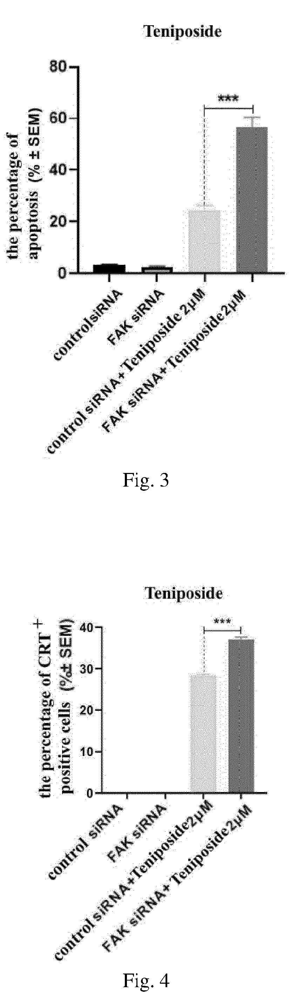

MDA-MB-231 cells were transfected with control siRNA or FAK siRNA at a final concentration of 50 nM, respectively. After the transfection for 24 hours, the cells were treated with 2 µM Teniposide for 48 hours. The cells were stained using Annexin V kit and detected by flow cytometry. The early and late apoptosis values were counted, compared with those of the DMSO control group, and plotted using Graphpad 8.0. The results showed that compared with the control group, the apoptosis of the FAK silencing group was significantly enhanced after Teniposide treatment, as shown in Fig. 3.

### 2. FAK silencing combined with Teniposide significantly upregulated the release and exposure of the ICD target Calreticulin

MDA-MB-231 cells were transfected with control siRNA or FAK siRNA at a final concentration of 50 nM, respectively. After the transfection for 24 hours, the cells were treated with 2 µM Teniposide for 48 hours. The cells were fluorescent stained with Calreticulin antibody, and the staining results were analyzed by flow cytometry. FlowJo software result statistics showed that after FAK silencing combined with Teniposide, the release and exposure of Calreticulin were significantly enhanced, as shown in Fig. 4.

### Example 3: Synergistic effect of IN10018 and Etoposide in colon cancer CT26 cells

CT26 cells were cultured with RPMI-1640 (Shanghai Basalmedia, catalog number: L210KJ, batch number: F210916) + 10% FBS (Gibco, catalog number: 10099-141c, batch number: 2158737cp), and passaged twice. When the cells were in good condition, they were placed in a 96-well plate, 3000 cells/well. After the cells were spread for 24 hours, the culture medium containing Etoposide was added. 10 drug concentrations were set. The first concentration was 30 µM, which was serially diluted 5-fold, and the last one was the control, with the drug concentration of 0. Each drug concentration was run in triplicate. At the same time, another group of cells was established with the same drug concentration as that in the above method. The difference was that: 5 µM IN10018 was added to each well, the drugs were mixed, and the plate was cultured in a 5% CO₂ incubator at 37 °C for 72 hours.

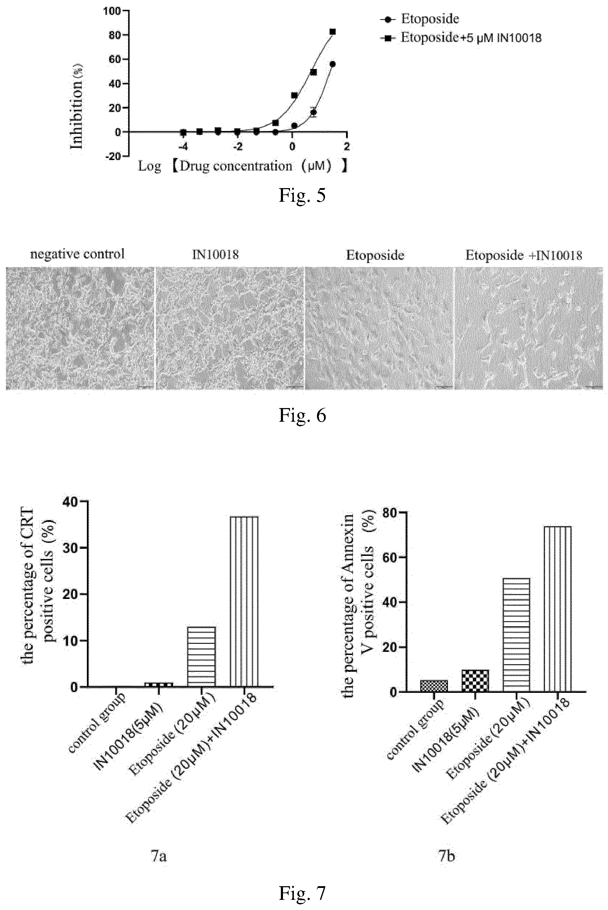

After 72 hours of drug action, the cells were observed under a microscope. 10 µl of CCK8 detection reagent (Cellorlab, classification number: CX001M, batch number: 2571100) was added to each well, and the plate was incubated in a 5% CO₂ incubator at 37 °C for 2-4 hours. Then the plate was read using microplate reader OD450 (chemiluminescence method). The analysis results showed that the IC50 of the Etoposide (source: MCE, classification number: 33419-42-0, batch number: 113741) group was 24.43 µM; and the IC50 of the Etoposide + 5 µM IN10018 group was 4.86 µM. The IC50 of the group containing IN10018 was significantly lower than that of the group without IN10018, indicating that the efficacy of the two-drug combination treatment group was better than that of the single-drug treatment group, as shown in Fig. 5.

### Example 4: Study on IN10018 and Etoposide in colon cancer CT26 cells

CT26 cells (Institute of Cell Biology, Chinese Academy of Sciences) were cultured with RPMI-1640 (Shanghai Basalmedia, catalog number: L210KJ, batch number: F210916) + 10% FBS (Gibco, catalog number: 10099-141c, batch number: 2158737cp), and passaged twice. When the cells were in good condition, they were placed in a 24-well plate. After 24 hours of cell spreading, four groups were set up. The first group was the control group, in which culture medium was added; the second group was IN10018 at a concentration of 5µM; the third group was Etoposide (source: MCE, classification number: 33419-42-0, batch number: 113741) with a concentration of 20 µM; and the fourth group was a combination group of IN10018 (5 µM) and Etoposide (20 µM). The drugs were mixed and cultured in a 5 % CO₂ incubator at 37°C for 48 hours.

After 48 hours of drug action, the cells were observed and photographed under a microscope. The photos were saved. Then the cells were collected for flow cytometry, and the cells were washed twice with flow buffer (PBS + 2% FBS). 0.5 µl of AF647 anti-Calreticulin antibody (abcam, catalog number: ab196159, batch number: CR33676773) was added to each well, and fully mixed. The cells were incubated at 4 °C for 20 minutes in the dark. After 20 minutes, the cells were washed twice with flow buffer (PBS + 2% FBS). Cell apoptosis detection kit (Beyotime, catalog number: CL062L, batch number: 021921210811) was used. 195 µl of Annexin-V-FITC binding solution was added, and the cells were gently mixed. 5 µl of annexin-V-FITC antibody was added, and the mixture was gently mixed. 10 µl of PI dye was added and gently mixed. The mixture was incubated at room temperature in the dark for 15 minutes, and then analyzed on a flow cytometer.

The cells were observed under a microscope. The cell status of the Etoposide single-drug group and the two-drug combination group were poor, wherein the two-drug combination group was the worst with more cell death. The cell status of the control group and IN10018 group were good. The results showed that the CRT positive rate and Annexin V positive rate of the two-drug combination group were higher than those of the single-drug group, as shown in Fig. 6, and Fig. 7.

### Example 5: Study on the Induction of immunogenic cell death targets by Irinotecan and IN10018 in mouse colon cancer CT26 cells in vitro

The grouping information is shown in Table 1.

**Table 1: Grouping scheme for in vitro efficacy assays**

| No. | Drug | Cell | Staining method |
|---|---|---|---|
| 1 | Control group | CT26 | CRT/Annexin-V/PI |
| 2 | IN10018(5 µM) | | |
| 3 | Irinotecan (126.76 µM) | | |
| 4 | Irinotecan (126.76 µM) + IN10018 (5 µM) | | |

The information of the tested compounds is shown in Table 2.

**Table 2: Information of the compounds to be tested**

| **Name** | Irinotecan | | IN10018 |
|---|---|---|---|
| **Supplier** | SuperLan Chemical Co., Ltd. | | Synthesized according to the method in patent WO2010058032 |
| **Batch number** | 202110 | | |
| **MW** | 623.14 | | 588.56 |
| **FW** | | | 738.65 |
| | | | |

| **Characteristics** | | White powder | White powder |
|---|---|---|---|
| **Solvent** | | DMSO | DMSO |
| **Storage conditions** | | 4°C | Room temperature |

The main reagents used in the assay are shown in Table 3.

**Table 3: Main reagents for the assay**

| **Reagent name** | **Manufacturer** | **Article number** | **Batch number** |
|---|---|---|---|
| DPBS | Shanghai Basalmedia | B210KJ | B210902 |
| RPMI-1640 | Shanghai Basalmedia | L210KJ | E211006 |
| FBS | GIBCO | 10099-141c | 2158737cp |
| Trypsin | Shanghai Basalmedia | S310KJ | C210903 |
| Annexin V-Apoptosis Detection Kit | Beyotime | C1062L | 021921210811 |
| AF647 Anti-Calreticulin Antibody | Abcam | ab196159 | CR33676773 |

### Assay methods and steps

### Cell culture

CT26 cells were maintained and passaged by InxMed (Nanjing) Co., Ltd. Cells were cultured in monolayer in vitro under the following conditions: RPMI-1640 medium with 10% fetal bovine serum, in a 37 °C, 5% CO₂ incubator. Cells were routinely digested and passaged using trypsin two to three times a week. When the cells were in the exponential growth phase and the adherent confluence reached 80%-90%, the cells were harvested and plated.

### Cell plating

CT26 cells were digested with trypsin, and then the cells were collected and counted. Based on the counting results, the cells were diluted with the corresponding complete culture medium to a concentration of 50,000 cells per milliliter. Then the cells were plated in a 24-well cell culture plate with 1 ml of cell suspension, i.e., 50,000 cells, per well. After plating, the cells were cultured in a 37 °C, 5% CO₂ incubator.

### Adding test compound

After 24 hours of plating, the test compounds Irinotecan and IN10018 were added to different wells. The test compounds were uniformly prepared and packaged before (dissolved in DMSO). The packaging volume was 50 µL/vial. They were stored at -20 °C in the dark. In this assay, one of the vials was taken out for drug addition and detection. The groups and drug concentrations are shown in Table 4.

**Table 4: Drug groups and dosing concentrations**

| **No.** | **Drug** |
|---|---|
| 1 | Medium |
| 2 | IN10018 (5 µM) |
| 3 | Irinotecan (126.76 µM) |
| 4 | Irinotecan (126.76 µM) + IN10018 (5 µM) |

### Collecting cells for flow cytometry:

The cells were washed twice with flow buffer (DPBS + 2% FBS). 0.5 µL of AF647 Anti-Calreticulin Antibody was added to each well, and mixed well. The cells were incubated at 4°C in the dark for 20 min. After washing once with flow buffer, 195 µL of Annexin-V-FITC binding solution was added, and the cells were gently mixed well. 5 µL of Annexin-V-FITC antibody was added. After further mixing, 10 µL of PI dye was added and mixed well. The cells were incubated at room temperature in the dark for 15 min. The cells were then subjected to flow cytometry.

### Data analysis

After the assay, the cell positive rate was analyzed by Flowjo (V10) software.

### Assay results

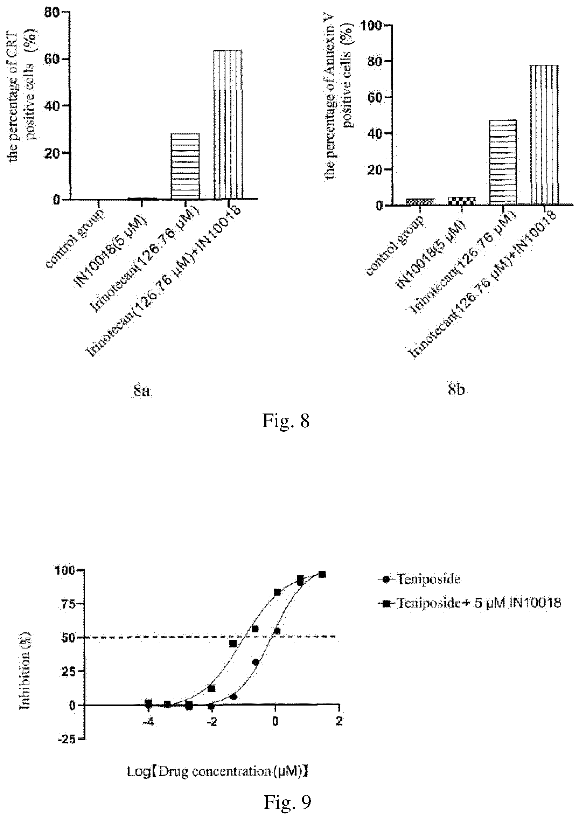

After 48 hours of drug action, the flow cytometric analysis results showed that the CRT positive rate and Annexin V positive rate of the combined drug group were significantly higher than those of the single-drug group, as shown in Fig. 8.

### Example 6: Study on the in vitro proliferation inhibitory activity of Teniposide and IN10018 on mouse colon cancer CT 26 cells

### Assay design

The grouping information is shown in Table 5.

**Table 5: Grouping scheme for cell proliferation inhibition test**

| **No.** | **Test drug** |
|---|---|
| 1 | Teniposide |
| 2 | Teniposide + IN10018 (5 µM) |

The information of the tested compounds is shown in Table 6.

**Table 6:**

| **Name** | Teniposide | IN10018 |
|---|---|---|
| **Supplier** | MCE | InxMed (Shanghai) Co., Ltd. |
| **Batch Number** | 24543 | Synthesized according to the method in patent WO2010058032 |
| **MW** | 656.65 | 588.56 |
| **FW** | | 738.65 |
| **Characteristics** | White powder | White powder |
| **Solvent** | DMSO | DPBS |
| **Storage conditions** | 4 °C | Normal temperature |

The main reagent information is shown in Table 7.

**Table 7:**

| **Reagent name** | **Manufacturer** | **Article number** | **Batch number** |
|---|---|---|---|
| DPBS | Shanghai Basalmedia | B210KJ | B210902 |
| RPMI-1640 | Shanghai Basalmedia | L210KJ | F210916 |
| FBS | GIBCO | 10099-141c | 2158737cp |
| Trypsin | Shanghai Basalmedia | S310KJ | C210903 |
| CCK-8 Kit | Service Bio | G4103-5ml | CR2112050 |

### Assay methods and steps:

### Cell culture:

CT26 cells were resuscitated, maintained, and passaged by InxMed (Nanjing) Co., Ltd. Cells were cultured in monolayer in vitro under the following conditions: RPMI-1640 medium with 10% fetal bovine serum, 37°C, 5% CO₂. Cells were routinely digested and passaged using trypsin two to three times a week. When the cells were in the exponential growth phase and the adherent confluence reached 80%-90%, the cells were harvested and plated.

### Cell plating

CT26 cells were digested with trypsin, and then the cells were collected and counted. Based on the counting results, the cells were diluted with RPMI 1640 + 10 % FBS to a concentration of 30,000 cells per milliliter. Then the cells were plated in a 96-well flat-bottom cell culture plate with 0.1 ml of cell suspension, i.e., 30,000 cells, per well. After plating, the cells were cultured in a 37 °C, 5% CO₂ incubator.

### Adding test compound

After 24 hours of plating, the test compound Teniposide was added to different wells of the cell plate. The test compound was uniformly prepared and packaged before the assay. In brief, the drug was dissolved in DMSO to 10 mM, the packaging volume was 50 µL/vial, and the drug was stored at -20 °C in the dark. In this assay, one of the vials was taken out for drug addition and treatment. The groups and drug concentrations are shown in Table 8.

**Table 8: Drug groups and dosing concentrations**

| No. | Test drug |
|---|---|
| 1 | Teniposide (maximum concentration 30 µM, 5-fold serial dilution, 10 action concentrations) |
| 2 | Teniposide (maximum concentration 30 µM, 5-fold serial dilution, 10 action concentrations) + IN10018 (5 µM) |

Ten drug action concentrations were designed for Teniposide, the last of which was 0, and was set as the negative control group.

After drug addition, the drugs were gently mixed and the cells were cultured in a 37 °C, 5% CO₂ incubator.

### Adding CCK-8 detection reagent and reading the plate:

After 72 hours of drug action, 10 µL of CCK-8 detection reagent was added to each well of the cell plate using a multi-channel pipette, and then the cell plate was incubated in a 37 °C, 5% CO₂ incubator for another 4 hours. Finally, the absorbance of each well at 450 nm was measured using an ELISA reader.

### Data Analysis:

After the assay, GraphPad Prism 8 software was used to analyze the inhibition percentage of the test drugs on the cells.

### Inhibition percentage calculation:

Inhibition percentage = {[A(0 drug addition)-A(blank)] -- [A(drug addition) - A(blank)]}/[A(0 drug addition)-A(blank)] × 100 %
A (drug addition): absorbance value of the wells with cells, CCK-8 solution and drug solution
A (blank): absorbance value of the wells with culture medium and CCK-8 solution but no cells
A (0 drug addition): absorbance value of the wells with cells and CCK-8 solution but no drug solution

### Assay conclusion:

This assay evaluated the inhibitory effects of Teniposide alone and in combination with IN10018 on the proliferation of CT26 cells in vitro. The relevant test results of each group after 72 hours of drug action are shown in Fig. 9. The IC50 value of the combination group of Teniposide and IN10018 was 0.09799 µM, which was lower than the IC50 value of the single drug group (0.8179 µM), so the combination group had a stronger in vitro inhibitory effect on cancer cell growth.

### Example 7: Study on the induction of immunogenic cell death targets by Teniposide and IN10018 in mouse colon cancer CT26 cells in vitro

The assay groups are shown in Table 9.

**Table 9:**

| No. | drug | cell | Staining method |
|---|---|---|---|
| 1 | Negative control | CT26 | |
| 2 | IN10018 (5 µM) | | 1.CRT/Annexin-V/PI |
| 3 | Teniposide (4.8 µM) | | 2.GRP94 |
| 4 | Teniposide (4.8 µM) + IN10018 (5 µM) | | |

The compound information is shown in Table 10:

**Table 10.**

| Name | Teniposide | IN10018 |
|---|---|---|
| Supplier | MCE | Synthesized according to the method in patent WO2010058032 |
| Batch Number | 24543 | |
| MW | 656.65 | 588.56 |
| FW | N/A | 738.65 |
| Characteristics | White powder | White powder |
| Solvent | DMSO | DPBS |
| Storage conditions | 4°C | Normal temperature |

The compound information is shown in Table 11.

**Table 11:**

| **Reagent name** | **Manufacturer** | **Article number** | **Batch number** |
|---|---|---|---|
| DPBS | Shanghai Basalmedia | B210KJ | B210902 |
| RPMI-1640 | Shanghai Basalmedia | L210KJ | F210916 |
| FBS | GIBCO | 10099-141c | 2158737cp |
| Trypsin | Shanghai Basalmedia | S310KJ | C210903 |
| Annexin V-Apoptosis Detection Kit | Beyotime | C1062L | 021921210811 |
| AF647 Anti-Calreticulin Antibody | Abcam | ab196159 | CR33676773 |

### Cell culture

CT26 cells were maintained and passaged by InxMed (Nanjing) Co., Ltd. Cells were cultured in monolayer in vitro under the following conditions: RPMI-1640 medium with 10% fetal bovine serum in a 37°C, 5% CO₂ incubator. Cells were routinely digested and passaged using trypsin two to three times a week. When the cells were in the exponential growth phase and the adherent confluence reached 80%-90%, the cells were harvested and plated.

### Cell plating

CT26 cells were digested with trypsin, and then the cells were collected and counted. Based on the counting results, the cells were diluted with RPMI1640 + 10% FBS to a concentration of 50,000 cells per milliliter. Then the cells were plated in a 12-well cell culture plate with 2 ml of cell suspension, i.e., 100,000 cells, per well.

After plating, the cells were cultured in a 37 °C, 5% CO₂ incubator.

### Adding test compound

After 24 hours of plating, the test compound Teniposide was added to different wells. The test compound was uniformly prepared and packaged before, dissolved in DMSO with a concentration of 10 mM, and the packaging volume was 50 µL/vial. They were stored at -20 °C in the dark. In this assay, one of the vials was taken out for drug addition and detection. The groups and drug concentrations are shown in Table 9.

After 48 hours of drug action, the cells were photographed using a microscope, then trypsinized and collected for flow cytometry staining.

The cells were washed twice with flow buffer (DPBS + 2% FBS). Each group of cells was divided into two equal parts. In one part, 0.5 µL of AF647 Anti-Calreticulin Antibody was added to each well, and mixed well. The cells were incubated at 4°C in the dark for 20 min. After washing once with flow buffer, 195 µL of Annexin-V-FITC binding solution was added, and the cells were gently mixed well. 5 µL of Annexin-V-FITC antibody was added. After further mixing, 10 µL of PI dye was added and mixed well. The cells were incubated at room temperature in the dark for 15 min. The cells were then subjected to flow cytometry.

### Data analysis

After the assay, the cell positive rate was analyzed by Flowjo (V10) software.

### Assay results:

This assay evaluated the effects of Teniposide alone and in combination with IN10018 on inducing the expression of immune cell death targets in CT26 cells under in vitro conditions.

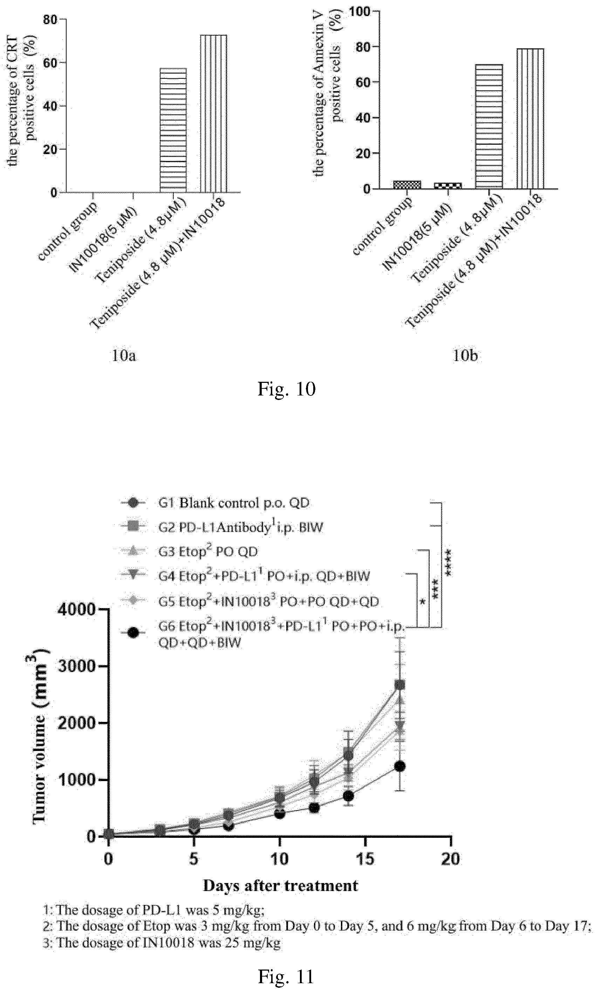

The relevant test results of each group after 48 hours of drug action are shown in Fig. 10. The cell status was observed under a microscope. The cell death in the Etoposide single drug group was obvious, and the cell death in the combined drug group was the most significant. The cell viability in the negative control group and IN10018 group were better. Flow cytometry analysis results showed that the positive rates of CRT and Annexin V in the combined drug group were significantly higher than those in the single drug group.

Example 8: In vivo antitumor efficacy study of the combination therapy between Etoposide, IN10018 and anti-mouse PD-L1 antibody in a subcutaneous allograft tumor model of colon cancer MC38 cells in C57BL/6 mice

Grouping and dosing information are shown in Table 12.

**Table 12: Animal groups and dosing regimens for in vivo efficacy assays**

| **Group** | **N¹** | **Compound to be tested** | **Dosage (mg/kg)** | **Dosing volume parameters (mL/kg)²** | **Route of administration** | **Dosing frequency** |
|---|---|---|---|---|---|---|
| 1 | 6 | Control group | N/A | 10 | PO | QD x 17 |
| 2 | 6 | Anti-mouse PD-L1 antibody | 5 | 10 | i.p. | BIW x 17 |
| 3 | 6 | Etoposide³ | 3 | 10 | PO | QD x 17 |
| 4 | 6 | Etoposide³ + anti-mouse PD-L1 antibody | 3 + 5 | 10 | PO + i.p. | QD x 17 + BIW x 17 |
| 5 | 6 | Etoposide³ +IN10018 | 3 + 25 | 10 | PO + PO | QD x 17 + QD x 17 |
| 6 | 6 | Etoposide³ + IN10018 + anti-mouse PD-L1 antibody | 3 + 25 + 5 | 10 | PO + PO + i.p. | QD x 17 + QD x 17 + BIW x 17 |

### Note:

1. N: number of mice in each group;
2. Dosing volume: 10 mL/kg based on the mouse body weight. If the body weight dropped by more than 15%, the administration of the animals would be stopped; When the body weight recovered to 90%, the administration would be resumed.
3. The Etoposide dose was increased to 6 mg/kg on the 6th day after grouping and dosing;

### Cell culture:

Colorectal cancer cells MC38 (from Nanjing Cobioer Biotechnology Co., Ltd., article number: CBP60825) were maintained and passaged by InxMed (Nanjing) Co., Ltd. Cells were cultured in monolayer in vitro under the following conditions: DMEM medium with 10% fetal bovine serum, in a 37°C, 5% CO₂ incubator. Cells were routinely digested and passaged two to three times a week using trypsin-EDTA. When the cells were in the exponential growth phase and the adherent confluence reached 80%-90%, the cells were harvested, counted, and then inoculated.

### Cell inoculating and grouping:

0.1 mL of cell suspension containing 2×10⁵ cells was subcutaneously inoculated on the right back of each mouse. When the tumor volume reached about 50 mm³ (9th day after cell inoculation), the mice were randomly divided into groups and administered according to the tumor volume. The preparation of the test substance and control solvent is shown in Table 13.

**Table 13: Test substance and control solvent preparation**

| **Drug name** | **Dosage (mg/kg)** | **Concentr ation (mg/ml)** | **Formulation process** | **Storage conditions and preparation frequency** |
|---|---|---|---|---|
| Control group | N/A | N/A | 1.5 ml of DMSO, 12 ml of PEG300, 1.5 ml of Tween-80 and 15 ml of PBS were pipetted sequentially. The mixture was vortexed. | Stored at 4 °C. |
| Etoposide | 3 | 0.3 | 12.0 mg of Etoposide powder was weighed and 2.0 ml of DMSO was added. The mixture was mixed by ultrasonication. 16 ml of PEG-300, 2.0 ml of Tween-80 and 20 ml of DPBS were added in sequence. The mixture was mixed well by pipetting to obtain a clear solution. | Stored at 4 °C, and prepared once every 7 days. |
| Etoposide | 6 | 0.6 | 24.0 mg of Etoposide powder was weighted and 2.0 ml of DMSO was added. The mixture was mixed by ultrasonication. 16 ml of PEG-300, 2.0 ml of Tween-80 and 20 ml of DPBS were added in sequence. The mixture was mixed well by pipetting to obtain a clear solution. | Stored at 4 °C, and prepared once every 7 days. |
| IN10018 | 25 | 2.5 | 30 mg of IN10018 was weighed and diluted by 12 mL of DPBS. The mixture was vortexed well to obtain a clear solution. | Stored at 4 °C and prepared once every 4 days. |
| mPD-L1 | 5 | 0.5 | 0.31 mL of PDL1 antibody (7.29 mg/mL) was pipetted and diluted by 4.19 mL of DPBS. The mixture was mixed well to obtain a clear solution. | Stored at 4 °C and used immediately after preparation. |

### Daily observation of assay animals

The formulation and any modifications of this assay protocol had been assessed and approved by the IACUC of ArrayBridge. The use and welfare of assay animals were carried out in accordance with AAALAC regulations. The health status and mortality of animals were monitored daily, with routine checks including observation of tumor growth and the impact of drug treatment on the animals' daily behavior, such as activity, food and water intake (estimated by visual inspection), changes in body weight, physical signs, or any other abnormalities. The number of animal deaths and side effects in each group were recorded based on the number of animals in each group.

### Assay termination

If the animal's health condition continued to deteriorate, or if the tumor volume exceeded 3,000 mm³, or there was a serious disease or pain, euthanasia should be carried out. In the following cases, the veterinarian was notified and euthanasia was performed: obvious weight loss, i.e., weight loss greater than 20%; inability to freely eat and drink; the average tumor volume of the control group reached 3,000 mm³, the assay was terminated. Animals showed the following clinical signs and continued to deteriorate: piloerection, hunched back, pale ears, nose, eyes, or feet, rapid breathing, convulsions, continuous diarrhea, dehydration, slow movement, vocalization.

### Tumor measurements and assay parameters

The assay index was to examine whether the tumor growth was inhibited, delayed, or cured. Tumor diameters were measured three times a week using calipers. The tumor volume was calculated using the formula: V = 0.5 × *a* × *b*², where *a* and *b* represent the long and short diameters of the tumor, respectively.

The anti-tumor efficacy of the compound was evaluated by TGI (%), which reflected the tumor growth inhibition rate. Based on the tumor volume on the first day after grouping, the tumor growth inhibition rate TGI (%) was calculated according to the following formula: TGI (%) = [1 - (average tumor volume of an administration group - average tumor volume of the administration group at the beginning of treatment) / (average tumor volume of the solvent control group - average tumor volume of the solvent control group at the beginning of treatment)] × 100%.

### Statistical analysis

Statistical analysis was based on tumor volume at the end of the assay using Prism Graphpad software. Comparisons between multiple groups were analyzed using Two-way ANOVA and Fisher's LSD test. A P-value of less than 0.05 was considered statistically significant.

### Assay results:

### 1. In vivo efficacy study of the combination therapy between the test substance Etoposide, IN10018, and anti-mouse PD-L1 antibody in a subcutaneous allograft tumor model of mouse colorectal cancer MC38 cells in C57BL/6 mice

After cell inoculation, tumor growth was observed every day. On the 9th day after inoculation, mice were grouped based on tumor volume. The average tumor volume at enrollment was approximately 50 mm³. Due to tumor burden, animals in the control group were euthanized on the 26th day after inoculation, i.e., the 17th day after administration according to the group, and the entire assay was over.

On the 17th day after administration according to the group, the tumor volume of the control group was 2670.9±1438.9 mm³. The tumor volumes of the PD-L1 antibody (5 mg/kg) and Etoposide (3 mg/kg) monotherapy groups were 2677.7±2029.0 mm³ and 2420.2±1377.3 mm³, respectively; the tumor volumes of the Etoposide + PD-L1 antibody (3+5 mg/kg) and Etoposide + IN10018 (3+25 mg/kg) combination therapy groups were 1947.6±539.7 mm ³ and 1865.0±839.9 mm³, respectively; the tumor volume of the Etoposide + IN10018 + PD-L1 antibody (3+25+5 mg/kg) triple therapy group was 1245.6±1064.7 mm³. Compared with the control group, the overall tumor volume showed that the tumor inhibition rates (TGI) of the PD-L1 antibody (5 mg/kg) and Etoposide (3 mg/kg) monotherapy groups were -0.2% (p = 0.9827) and 9.5% (p = 0.4487), respectively; the tumor inhibition rates (TGI) of the Etoposide + PD-L1 antibody (3 + 5 mg/kg) and Etoposide + IN10018 (3 + 25 mg/kg) combination therapy groups were 27.6% (p = 0.0295) and 30.7% (p = 0.0111), respectively; the tumor inhibition rate (TGI) of the Etoposide + IN10018 + PD-L1 antibody (3 + 25 + 5 mg/kg) triplet therapy group was 54.4% (p < 0.0001).

The overall tumor volume was compared with the Etoposide + IN10018 + PD-L1 antibody (3 + 25 + 5 mg/kg) triple drug combination group for statistical analysis. The P values of the control group, PD-L1 antibody (5 mg/kg) and Etoposide (3 mg/kg) monotherapy group were p < 0.0001, p < 0.0001 and p = 0.0005, respectively; the P values of the Etoposide + PD-L1 antibody (3 + 5 mg/kg) and Etoposide + IN10018 (3 + 25 mg/kg) dual drug combination therapy group were p = 0.0346 and p = 0.0504, respectively, as shown in Table 14. The tumor volume of each dose group at different time periods is shown in Fig. 11. Compared with the blank control group, the average tumor volume of the PD-L1 antibody (5 mg/kg) and Etoposide (3 mg/kg) monotherapy groups was very close to that of the control group, and no inhibitory effect on tumor growth was shown; while the Etoposide + PD-L1 antibody (3+5 mg/kg) and Etoposide + IN10018 (3+25 mg/kg) combination therapy groups and the Etoposide + IN10018 + PD-L1 antibody (3+25+5 mg/kg) triple therapy group all had significant tumor growth inhibitory effects, which were statistically different from the control group. Comprehensively considering the entire dosing cycle, the tumor volume of the Etoposide + IN10018 + PD-L1 antibody (3+25+5 mg/kg) triple combination group was always smaller than that of the other treatment groups, and there were statistical differences compared with the PD-L1 antibody (5 mg/kg) and Etoposide (3 mg/kg) monotherapy groups and the Etoposide + PD-L1 antibody (3+5 mg/kg) two-drug combination treatment group. Although there was no statistical difference between the Etoposide + IN10018 + PD-L1 antibody (3+25+5 mg/kg) triple combination group and the Etoposide + IN10018 (3+25 mg/kg) two-drug combination treatment group, the average tumor volume of the Etoposide + IN10018 + PD-L1 antibody (3+25+5 mg/kg) triple combination group was always relatively small, which also showed that the Etoposide + IN10018 + PD-L1 antibody (3+25+5 mg/kg) triple combination group had a better effect in inhibiting tumor growth.

**Table 14: Evaluation of the tumor inhibition effect of the test substance on the C57BL/6 mouse transplanted tumor model of mouse colon cancer MC38 cells (based on the data on the 17th day after group administration)**

| **Group** | **Tumor volume on day 0 (mm³)¹** | **Tumor volume on day 17 (mm³)¹** | **TGI (%)²** | **P value³** | **P value⁴** |
|---|---|---|---|---|---|
| Control group | 49.3 ± 13.9 | 2670.9 ± 1438.9 | / | / | p < 0.0001 **** |
| PD-L1 Antibodies | 50.7 ± 18.6 | 2677.7 ± 2029.0 | -0.2 | 0.9827 | p < 0.0001 **** |
| Etoposide | 48.2 ± 10.2 | 2420.2 ± 1377.3 | 9.5 | 0.4487 | 0.0005 *** |
| Etoposide + PD-L1 antibody | 49.2 ± 12.5 | 1947.6 ± 539.7 | 27.6 | 0.0295 * | 0.0346 * |
| Etoposide + IN10018 | 49.2 ± 12.3 | 1865.0 ± 839.9 | 30.7 | 0.0111 * | 0.0504 |
| Etoposide + IN10018 + PD-L1 antibody | 49.0 ± 11.6 | 1245.6 ± 1064.7 | 54.4 | p < 0.0001 **** | / |

### Note:

1. Calculated according to the number of days after group administration, the data are mean ± standard deviation (mean ± SD);
2. TGI (%) = [1-(T₁₇ -T₀)/(V₁₇ - V₀)] ×100%;
3. *: p < 0.05, vs. control group, Two-way ANOVA;
4. *: p<0.05, vs. Etoposide+IN10018+PD-L1 antibody (3+25+5 mg/kg) group, Two-way ANOVA;

### 2. Study on the changes in body weight and clinical status of the combination therapy between the test substances etoposide, IN10018 and anti-mouse PD-L1 antibody in the MC38 mouse colorectal cancer cell subcutaneous allograft tumor model of C57BL/6 mice

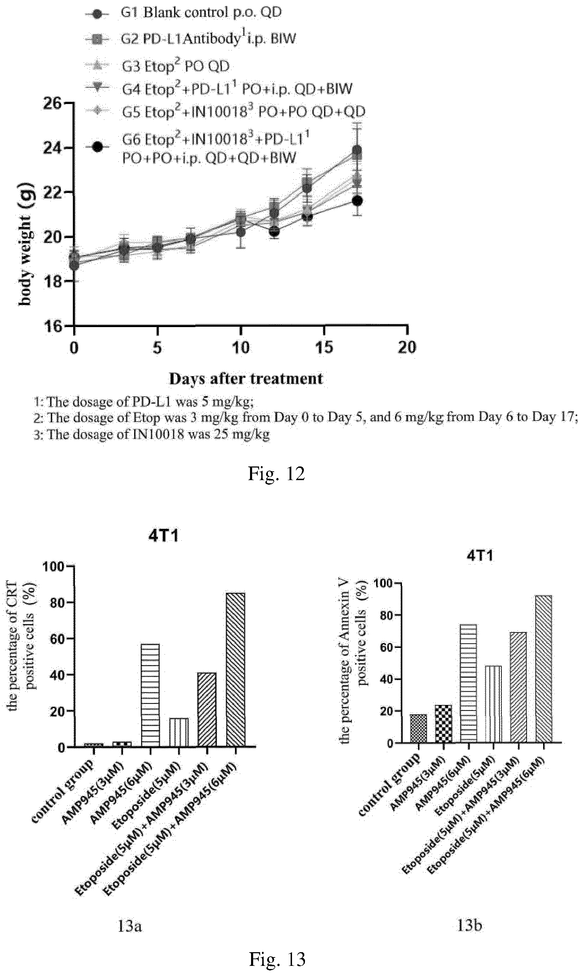

The assay was carried out according to the dosing regimen. During the assay, the animals' activities such as eating and drinking were observed every day, and the body weight of the animals was recorded three times a week. After 17 days of group administration, the average body weight of the control group changed from 18.7g on the day of group administration (Day 0) to 23.9g, with a body weight growth rate of 28.8%; the average body weight of the PD-L1 antibody (5mg/kg) and Etoposide (3 mg/kg) monotherapy groups changed from 18.8g and 19.1g on Day 0 to 23.6g and 22.8g on Day 17, respectively, with body weight change rates of 25.8% and 19.3%, respectively; the average body weight of the Etoposide + PD-L1 antibody (3+5mg/kg) and Etoposide + IN10018 (3+25mg/kg) combination therapy groups changed from 19.1g and 18.9g on Day 0 to 22.3g and 22.5g on Day 17, respectively, with body weight change rates of 17.2% and 19.0%, respectively; The average body weight of the Etoposide + IN10018+ PD-L1 antibody (3+25+5mg/kg) three-drug combination group changed from 19.1g on Day 0 to 21.6g on Day 17, with a weight change rate of 13.3%. During the entire dosing cycle, one mouse in each of the Etoposide (3 mg/kg) monotherapy group and the Etoposide + PD-L1 antibody (3+5 mg/kg) two-drug combination therapy groups died on the 12th and 17th days after group administration due to tumor rupture. The animals in the remaining groups did not lose significant weight and were in good condition during the entire dosing cycle, showing tolerance to different dosing methods. See Table 15 for details. The weight changes of each dose group at different time periods are shown in Fig. 12. This shows that the animals are tolerant to the three-drug combination of Etoposide + IN10018 + PD-L1 antibody (3+25+5 mg/kg).

**Table 15: Evaluation of the test substance on the body weight change of the C57BL/6 mouse allograft tumor model of mouse colon cancer MC38 cells (based on the data on the 17th day after group administration)**

| **Group** | **Animal Survival ¹** | **Body weight on day 0 (g) ²** | **Body weight on day 17 (g) ²** | **Body weight change rate (%) ³** |
|---|---|---|---|---|
| Control group | 6/6 | 18.7 ± 1.7 | 23.9 ± 2.3 | 28.8 |
| PD-L1 Antibodies | 6/6 | 18.8 ± 0.5 | 23.6 ± 3.5 | 25.8 |
| Etoposide | 5/6 | 19.1 ± 1.1 | 22.8 ± 1.3 | 19.3 |
| Etoposide + PD-L1 antibody | 5/6 | 19.1 ± 0.4 | 22.3 ± 0.9 | 17.2 |
| Etoposide + IN10018 | 6/6 | 18.9 ± 0.7 | 22.5 ± 1.7 | 19.0 |
| Etoposide + IN10018 + PD-L1 antibody | 6/6 | 19.1 ± 0.7 | 21.6 ± 1.6 | 13.3 |

### Note:

1. Calculated according to the number of days after group administration, the number of animals surviving on day 0/ the number of animals surviving on day 17;
2. Data are mean ± standard deviation (mean ± SD);
3. Body weight change rate = [1-(W₁₇₋W₀)/W₀] * 100%;

### Example 9: Study on the induction of immunogenic cell death targets of mouse breast cancer 4T1 cells in vitro by Etoposide and AMP945

### Assay Materials:

### 1) Drugs used in this assay

Etoposide was provided by MCE, Lot No.: 113741
AMP945 was provided by MCE, Lot No.: 143253.

### 2) Antibodies used in this assay

Recombinant Alexa Fluor^{®} 647 fluorescent Anti-Calreticulin antibody (Abcam, Cat No.: ab196159, Lot No.: CR33676773). Annexin V-apoptosis detection kit (Beyotime, Cat No.: C1062L, Lot No.: 122221220706).

### Assay method:

4T1 cells (from Nanjing Cobioer Biotechnology Co., Ltd., article number: CBP60352) were cultured with RPMI-1640 (Shanghai Basalmedia, Cat No.: L210KJ, Lot No.: F210916)+10% FBS (Gibco, Cat No.: 10099-141c, Lot No.: 2158737cp), wherein the culture conditions were 37 °C and 5% CO₂. Trypsin was used for routine digestion and passage treatment two or three times a week. When the cells were in exponential growth period and the adherent confluence reached 80%-90%, the cells were collected and plated. 4T1 cells were digested with trypsin. The cells were then collected and counted. According to the counting results, the cells were diluted with RPMI-1640+10%FBS, and the dilution concentration was 50,000 cells per ml. Then, the cells were plated on 12-well culture plates, and 2 ml of cell suspension (100,000 cells) was laid in each well. After plating, the cells were cultured in 37 °C and 5% CO₂ incubator. After the cells were spread for 24 hours, six groups were set up, wherein the first group was a control group, and the culture medium was added; the second group was AMP945 with a concentration of 3µM; the third group was AMP945 with a concentration of 6 µM; the fourth group was Etoposide with a concentration of 5 µM; the fifth group was AMP945 (3 µM) combined with Etoposide (5 µM); the sixth group was AMP945 (6 µM) combined with Etoposide (5 µM). The drugs were mixed and the mixture was cultured in a 5%CO₂ incubator at 37°C for 48 hours.

### Assay result

After 48 hours of drug action, the cells were collected for flow cytometry. The cells were washed twice with flow buffer (PBS+2%FBS). 0.5 µl of AF647 anti-Calreticulin antibody (abcam) was added to each well, and mixed. The cells were incubated at 4°C in the dark. After 20 min of incubation, the flow buffer was added. Annexin V-Apoptosis Detection kit (Beyotime) was used. 195 µl of Annexin-V-FITC binding solution was added and mixed with the cells by pipetting. 5 µl of Annexin-V-FITC antibody was added, and gently mixed. Finally, 10 µl of PI dye was added and mixed, and the cells were incubated in the dark at room temperature for 15 min. The sample was sent to a flow cytometer for signal determination.

The results of flow cytometry analysis showed that the CRT positive rate and the Annexin-V positive rate in the two-drug combination group were significantly better than those in the single drug group and the control group, as shown in Fig. 13.

### Example 10: Study on the induction of immunogenic cell death targets of mouse breast cancer 4T1 cells in vitro by Irinotecan and AMP945

### Assay materials:

### 1) Drugs used in this assay

Irinotecan provided by Shanghai Chaolan Chemical Technology Center, Lot No.: 202110
AMP945 was provided by MCE, Lot No.: 143253.

### 2) Antibodies used in this assay

Recombinant Alexa Fluor^{®} 647 fluorescent Anti-Calreticulin antibody (Abcam, Cat No.: ab196159, Lot No.: CR33676773). Annexin V-apoptosis detection kit (Beyotime, Cat No.: C1062L, Lot No.: 122221220706).

### Assay method:

4T1 cells (from Nanjing Cobioer Biotechnology Co., Ltd., article number: CBP60352) were cultured with RPMI 1640 (Shanghai Basalmedia, Cat No.: L210KJ, Lot No.: F210916)+10%FBS (Gibco, Cat No.: 10099-141c, Lot No.: 2158737cp), wherein the culture conditions were 37 °C and 5% CO₂. Trypsin was used for routine digestion and passage treatment two or three times a week. When the cells were in exponential growth period and the adherent confluence reached 80%-90%, the cells were collected and plated. 4T1 cells were digested with trypsin. The cells were then collected and counted. According to the counting results, the cells were diluted with RPMI-1640+10%FBS, and the dilution concentration was 50,000 cells per ml. Then, the cells were plated on 12-well culture plates, and 2 ml of cell suspension (100,000 cells) was laid in each well. After plating, the cells were cultured in 37 °C and 5% CO₂ incubator. After the cells were spread for 24 hours, six groups were set up, wherein the first group was a control group, and the culture medium was added; the second group was AMP945 with a concentration of 3µM; the third group was AMP945 with a concentration of 6 µM; the fourth group was Irinotecan with a concentration of 120 µM; the fifth group was AMP945(3 µM) combined with Irinotecan (120 µM); the sixth group was AMP945(6 µM) combined with Irinotecan (120 µM). The drugs were mixed and the mixture was cultured in a 5%CO₂ incubator at 37°C for 48 hours.

### Assay result

After 48 hours of drug action, the cells were collected for flow cytometry. The cells were washed twice with flow buffer (PBS+2%FBS). 0.5 µl of AF647 anti-Calreticulin antibody (abcam) was added to each well, and mixed. The cells were incubated at 4°C in the dark. After 20 min of incubation, the flow buffer was added. Annexin-V Apoptosis Detection kit (Beyotime) was used. 195 µl of Annexin-V-FITC binding solution was added and mixed with the cells by pipetting. 5 µl of Annexin-V-FITC antibody was added, and gently mixed. Finally, 10 µl of PI dye was added and mixed, and the cells were incubated in the dark at room temperature for 15 min. The sample was sent to a flow cytometer for signal determination.

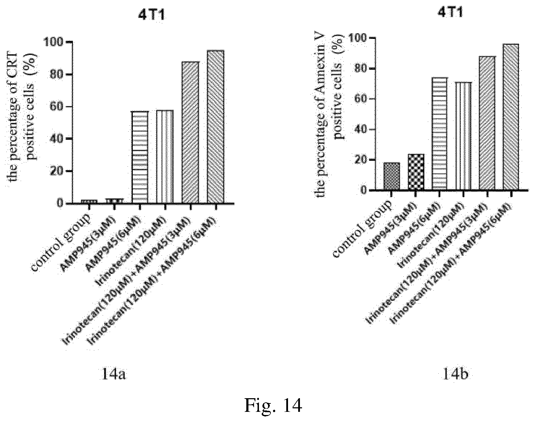

The results of flow cytometry analysis showed that the CRT positive rate and the Annexin-V positive rate in the two-drug combination group were significantly better than those in the single drug group and the control group, as shown in Fig. 14.

All references mentioned in the present disclosure are incorporated herein by reference in their entirety as if each document was listed separately. It should be understood that after reading the present disclosure, those skilled in the art can make various changes or modifications to the present disclosure, and these equivalents also fall within the scope defined by the claims of this application.

## Claims

1. Use of a FAK inhibitor, a topoisomerase inhibitor and an immune checkpoint inhibitor in the manufacture of a medicament for treating a tumor in a subject, wherein the topoisomerase inhibitor is not an anthracycline.

2. A pharmaceutical combination product of a FAK inhibitor, a topoisomerase inhibitor and an immune checkpoint inhibitor for use in treating a tumor in a subject, wherein the topoisomerase inhibitor is not an anthracycline.

3. A method for treating a tumor, the method comprising administering to a subject a therapeutically effective amount of a FAK inhibitor, a topoisomerase inhibitor, and an immune checkpoint inhibitor, wherein the topoisomerase inhibitor is not an anthracycline.

4. The use, the pharmaceutical combination product or the method according to any one of claims 1 to 3, wherein the FAK inhibitor and the topoisomerase inhibitor induce immunogenic cell death (ICD), wherein the topoisomerase inhibitor is not an anthracycline.

5. The use, the pharmaceutical combination product or the method according to any one of claims 1 to 4, wherein the FAK inhibitor is IN10018, Defactinib, GSK2256098, PF-00562271, VS-4718, APG-2449, AMP945, AMP 886 or a pharmaceutically acceptable salt thereof, alternatively, the FAK inhibitor is IN10018, Defactinib, AMP945 or a pharmaceutically acceptable salt thereof, yet alternatively, the FAK inhibitor is IN10018 or a pharmaceutically acceptable salt thereof, alternatively, the FAK inhibitor is IN10018 tartrate, wherein the IN10018 has a structure of:

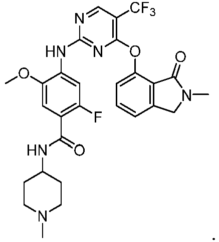

6. The use, the pharmaceutical combination product or the method according to any one of claims 1 to 5, wherein the topoisomerase inhibitor is a topoisomerase I inhibitor.

7. The use, the pharmaceutical combination product or the method according to claim 6, wherein the topoisomerase I inhibitor is Topotecan, Irinotecan, Belotecan, Cositecan, Exatecan (DX-8951), Indenoisoquinoline, Indotecan (LMP-400), Indimitecan (LMP-776), Simmitecan, Gimatecan (ST1481), EC-112002, PLX-038 (NK012), AR-67, or a pharmaceutically acceptable salt thereof.

8. The use, the pharmaceutical combination product or the method according to claim 7, wherein the topoisomerase I inhibitor is Irinotecan, or a pharmaceutically acceptable salt thereof.

9. The use, the pharmaceutical combination product or the method according to any one of claims 1 to 5, wherein the topoisomerase inhibitor is a topoisomerase II inhibitor.

10. The use, the pharmaceutical combination product or the method according to claim 9, wherein the topoisomerase II inhibitor is Etoposide, Teniposide, Sabarubicin, or a pharmaceutically acceptable salt thereof.

11. The use, the pharmaceutical combination product or the method according to claim 10, wherein the topoisomerase II inhibitor is Etoposide, Teniposide, or a pharmaceutically acceptable salt thereof.

12. The use, the pharmaceutical combination product or the method according to any one of claims 1 to 11, wherein the immune checkpoint inhibitor is an anti-PD-1/PD-L1 antibody, a PD-1/PD-L1 small molecule inhibitor or a TIGIT antibody.

13. The use, the pharmaceutical combination product or the method according to any one of claims 1 to 12, wherein the immune checkpoint inhibitor is an anti-PD-1/PD-L1 antibody, optionally, the anti-PD-1/PD-L1 antibody is Pembrolizumab, Tislelizumab, Nivolumab, Toripalimab, Atezolizumab, Durvalumab, Avelumab, Atezolizumab, Camrelizumab, Sintilimab, Cemiplimab, Envafolimab, BMS-936559, JS003, SHR-1316, GS-4224, AN-4005 or MX-10181.

14. The use, the pharmaceutical combination product or the method according to any one of claims 1 to 12, wherein the immune checkpoint inhibitor is a PD-1/PD-L1 small molecule inhibitor, optionally, the PD-1/PD-L1 small molecule inhibitor is INCB-086550, Lazertinib, IMMH-010, CA-170, ABSK043 or RRx-001.

15. The use, the pharmaceutical combination product or the method according to any one of claims 1 to 12, wherein the immune checkpoint inhibitor is a TIGIT antibody, optionally, the TIGIT antibody is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036 or IBI-321.

16. The use, the pharmaceutical combination product or the method according to any one of claims 1 to 5, wherein the FAK inhibitor is IN10018 or a pharmaceutically acceptable salt thereof; the topoisomerase inhibitor is Irinotecan or a pharmaceutically acceptable salt thereof; the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.

17. The use, the pharmaceutical combination product or the method according to any one of claims 1 to 5, wherein the FAK inhibitor is IN10018 or a pharmaceutically acceptable salt thereof; the topoisomerase inhibitor is Etoposide, Teniposide, or a pharmaceutically acceptable salt thereof; the immune checkpoint inhibitor is an anti-PD-1/PD-L1 antibody or a PD-1/PD-L1 small molecule inhibitor, alternatively, the immune checkpoint inhibitor is an anti-PD-1/PD-L1 antibody.

18. The use, the pharmaceutical combination product or the method according to any one of claims 1 to 17, wherein the FAK inhibitor, the topoisomerase inhibitor and the immune checkpoint inhibitor are administered simultaneously or sequentially to the subject.

19. The use, the pharmaceutical combination product or the method according to any one of claims 1 to 18, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian cancer, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma or pancreatic cancer.

20. The use, the pharmaceutical combination product or the method according to any one of claims 1 to 19, wherein the tumor is breast cancer or colon cancer (including colorectal cancer).

21. A kit or a pharmaceutically acceptable composition comprising:
(a) a FAK inhibitor;
(b) a topoisomerase inhibitor, wherein the topoisomerase inhibitor is not an anthracycline; and
(c) an immune checkpoint inhibitor.

22. The kit or the composition according to claim 21, wherein the FAK inhibitor and the topoisomerase inhibitor induce immunogenic cell death (ICD).

23. The kit or the composition according to any one of claims 21 to 22, wherein the FAK inhibitor is IN10018, Defactinib, GSK2256098, PF-00562271, VS-4718, APG-2449, AMP945, AMP886 or a pharmaceutically acceptable salt thereof, alternatively the FAK inhibitor is IN10018, Defactinib, AMP945 or a pharmaceutically acceptable salt thereof, yet alternatively, the FAK inhibitor is IN10018 or a pharmaceutically acceptable salt thereof, alternatively, the FAK inhibitor is IN10018 tartrate, wherein the IN10018 has a structure of:

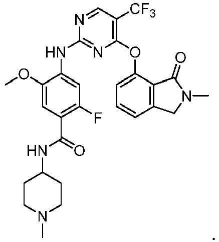

24. The kit or the composition according to any one of claims 21 to 23, wherein the topoisomerase inhibitor is a topoisomerase I inhibitor.

25. The kit or the composition according to claim 24, wherein the topoisomerase I inhibitor is Topotecan, Irinotecan, Belotecan, Cositecan, Exatecan (DX-8951), Indenoisoquinoline, Indotecan (LMP-400), Indimitecan (LMP-776), Simmitecan, Gimatecan (ST1481), EC-112002, PLX-038 (NK012), AR-67, or a pharmaceutically acceptable salt thereof.

26. The kit or the composition according to claim 25, wherein the topoisomerase I inhibitor is Irinotecan, or a pharmaceutically acceptable salt thereof.

27. The kit or the composition according to any one of claims 21-23, wherein the topoisomerase inhibitor is a topoisomerase II inhibitor.

28. The kit or the composition according to claim 27, wherein the topoisomerase II inhibitor is Etoposide, Teniposide, Sabarubicin, or a pharmaceutically acceptable salt thereof.

29. The kit or the composition according to claim 28, wherein the topoisomerase II inhibitor is Etoposide, Teniposide, or a pharmaceutically acceptable salt thereof.

30. The kit or the composition according to any one of claims 21-29, wherein the immune checkpoint inhibitor is an anti-PD-1/PD-L1 antibody, a PD-1/PD-L1 small molecule inhibitor, or a TIGIT antibody.

31. The kit or the composition according to any one of claims 21-30, wherein the immune checkpoint inhibitor is an anti-PD-1/PD-L1 antibody, optionally, the anti-PD-1/PD-L1 antibody is Pembrolizumab, Tislelizumab, Nivolumab, Toripalimab, Atezolizumab, Durvalumab, Avelumab, Atezolizumab, Camrelizumab, Sintilimab, Cemiplimab, Envafolimab, BMS-936559, JS003, SHR-1316, GS-4224, AN-4005 or MX-10181.

32. The kit or the composition according to any one of claims 21 to 30, wherein the immune checkpoint inhibitor is a PD-1/PD-L1 small molecule inhibitor, optionally, the PD-1/PD-L1 small molecule inhibitor is INCB-086550, Lazertinib, IMMH-010, CA-170, ABSK043 or RRx-001.

33. The kit or the composition according to any one of claims 21 to 30, wherein the immune checkpoint inhibitor is a TIGIT antibody, optionally, the TIGIT antibody is Ociperlimab (BGB-A1217), Vibostolimab, Domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036 or IBI-321.

34. The kit or the composition according to any one of claims 21 to 22, wherein the FAK inhibitor is IN10018 or a pharmaceutically acceptable salt thereof; the topoisomerase inhibitor is Irinotecan or a pharmaceutically acceptable salt thereof; the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.

35. The kit or the composition according to any one of claims 21 to 22, wherein the FAK inhibitor is IN10018 or a pharmaceutically acceptable salt thereof; the topoisomerase inhibitor is Etoposide, Teniposide, or a pharmaceutically acceptable salt thereof; the immune checkpoint inhibitor is an anti-PD-1/PD-L1 antibody or a PD-1/PD-L1 small molecule inhibitor, alternatively the immune checkpoint inhibitor is an anti-PD-1/PD-L1 antibody.

36. The kit or the composition according to any one of claims 21 to 35 for use as a medicament.

37. The kit or the composition according to any one of claims 21 to 36, wherein the medicament is used to treat a tumor, and the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian cancer, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma or pancreatic cancer.

38. The kit or the composition according to claim 37, wherein the tumor is breast cancer or colon cancer (including colorectal cancer).

39. A FAK inhibitor for use to enhance immunogenic cell death induced by a topoisomerase inhibitor in the treatment of a tumor, wherein the topoisomerase inhibitor is not an anthracycline.

40. The FAK inhibitor according to claim 39, wherein the FAK inhibitor is IN10018, Defactinib, GSK2256098, PF-00562271, VS-4718, APG-2449, AMP945, AMP 886 or a pharmaceutically acceptable salt thereof, alternatively the FAK inhibitor is IN10018, Defactinib, AMP945 or a pharmaceutically acceptable salt thereof, yet alternatively, the FAK inhibitor is IN10018 or a pharmaceutically acceptable salt thereof, alternatively the FAK inhibitor is IN10018 tartrate, and the IN10018 has a structure of:

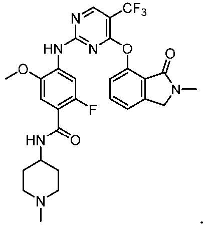

41. The FAK inhibitor according to claim 39 or 40, wherein the topoisomerase inhibitor is a topoisomerase I inhibitor.

42. The FAK inhibitor according to claim 41, wherein the topoisomerase I inhibitor is Topotecan, Irinotecan, Belotecan, Cositecan, Exatecan (DX-8951), Indenoisoquinoline, Indotecan (LMP-400), Indimitecan (LMP-776), Simmitecan, Gimatecan (ST1481), EC-112002, PLX-038 (NK012), AR-67, or a pharmaceutically acceptable salt thereof.

43. The FAK inhibitor according to claim 42, wherein the topoisomerase I inhibitor is Irinotecan, or a pharmaceutically acceptable salt thereof.

44. The FAK inhibitor according to claim 39 or 40, wherein the topoisomerase inhibitor is a topoisomerase II inhibitor.

45. The FAK inhibitor according to claim 44, wherein the topoisomerase II inhibitor is Etoposide, Teniposide, Sabarubicin, or a pharmaceutically acceptable salt thereof.

46. The FAK inhibitor according to claim 45, wherein the topoisomerase II inhibitor is Etoposide, Teniposide, or a pharmaceutically acceptable salt thereof.

47. The FAK inhibitor according to any one of claims 39 to 46, wherein the tumor is bladder cancer, breast cancer, cervical cancer, colon cancer (including colorectal cancer), esophageal cancer, esophageal squamous cell carcinoma, head and neck cancer, liver cancer, lung cancer (including small cell lung cancer and non-small cell lung cancer), melanoma, myeloma, rhabdomyosarcoma, inflammatory myofibroblastoma, neuroblastoma, pancreatic cancer, prostate cancer, kidney cancer, renal cell carcinoma, sarcoma (including osteosarcoma), skin cancer (including squamous cell carcinoma), gastric cancer, testicular cancer, thyroid cancer, uterine cancer, mesothelioma, cholangiocarcinoma, leiomyosarcoma, liposarcoma, nasopharyngeal carcinoma, neuroendocrine carcinoma, ovarian cancer, salivary adenocarcinoma, metastases caused by spindle cell carcinoma, anaplastic large cell lymphoma, undifferentiated thyroid cancer, non-Hodgkin's lymphoma, Hodgkin's lymphoma, glioma or hematologic malignancies such as acute myeloid leukemia (AML), acute lymphocytic leukemia (ALL), diffuse large B-cell lymphoma (DLBCL), follicular lymphoma (FL), chronic lymphocytic leukemia (CLL), or chronic myelogenous leukemia (CML); alternatively, the tumor is breast cancer, ovarian cancer, colon cancer (including colorectal cancer), lung cancer (including small cell lung cancer and non-small cell lung cancer), melanoma or pancreatic cancer.

48. The FAK inhibitor according to any one of claims 39 to 47, wherein the tumor is breast cancer or colon cancer (including colorectal cancer).
